# EUROPEAN PATENT SPECIFICATION

(11) **EP 2 021 508 B1**
(45) Date of publication and mention of the grant of the patent: **25.04.2012**
(21) Application number: 07762310.6
(22) Date of filing: 23.05.2007
(51) Int. Cl.: C12Q 1/68

(54) **AMBIENT TEMPERATURE STABLE KITS FOR MOLECULAR DIAGNOSTICS**
UMGEBUNGSTEMPERATURSTABILE KITS FÜR DIE MOLEKULARE DIAGNOSTIK
KITS STABLES À LA TEMPÉRATURE AMBIANTE POUR DES DIAGNOSTICS MOLÉCULAIRES

(30) Priority: 23.05.2006 US 802510 P
(43) Date of publication of application: 11.02.2009
(60) Divisional application: 10001187.3
(73) Proprietor: Molecular Detection, Inc., Berwyn, PA 19312 (US)
(72) Inventor: ARIELI, Boaz, 96756 Jerusalem (IL); SHKEDY, Fanny, Szafer, 96821 Jerusalem (IL); ROITMAN, Vered, 90000 Petach Tikvah (IL); BAR-AKIVA, Giora, 99745 Gizo (IL); GASSEL, Aryeh, 97234 Jerusalem (IL)
(74) Representative: O'Neill, Michelle
(86) International application number: PCT/US2007/069566
(87) International publication number: WO 2007/137291

(56) References cited:
- EP-A- 1 078 984
- WO-A-94/16107
- US-A- 5 641 864
- US-A- 5 861 251
- US-A- 6 153 412
- US-A- 6 153 412
- US-A1- 2002 173 016
- US-A1- 2003 119 042
- US-A1- 2004 072 167
- US-B1- 6 294 365
- US-B1- 6 294 365

## Description

### RELATED APPLICATIONS

This application claims priority to and the benefit of U.S. Provisional Application No. 60/802,510, titled Ambient temperature stable kits for molecular detection, to Boaz Arieli et al., filed May 23, 2006.

### FIELD OF THE INVENTION

The present invention generally relates to the field of molecular diagnostic kits and methods thereof. More specifically, the present invention relates to a solution mix that is hydration reduced and ambient temperature stabilized and can serve as a ready-to-use kit for pathogens identification and diagnosis of diseases from amplified nucleic acid samples utilizing polymerase chain reaction or quantitative polymerase chain reaction. The present invention also relates to methods for preparing such mixes and kits containing them.

### BACKGROUND OF THE INVENTION

Molecular diagnostics generally refers to an analysis of nucleic acids to determine the presence of infectious agents, inherited diseases, cancers or variations in the genetic profile of a patient that have been associated to susceptibility, severity, progression or responsiveness to therapy. Molecular diagnostic test procedures typically include in vitro amplification of a DNA sample. DNA polymerase chain reaction (hereinafter referred to as "PCR") and quantitative polymerase chain reaction (hereinafter referred to as "qPCR") are by far the most widely used methods of DNA amplification to date.

PCR allows a specific target sequence to be amplified exponentially to a factor of 106. PCR amplification involves two oligonucleotide primers that flank the DNA segment to be amplified and repeated cycles of heat denaturation of the DNA, annealing of the primers to their complementary sequences and extension of the annealed primers with a DNA polymerase (Kolmodin and Williams, 2000).

Quantitative PCR, sometimes referred to as "real-time PCR", utilizes the same amplification scheme as PCR, with two oligonucleotide primers flanking the DNA segment to be amplified. In qPCR, the reaction products are monitored as they are being formed. Monitoring may be "On-Line" or "Real-Time." Several methods can be used for real time monitoring, all of which rely on florescent labeling. One common method used in real time employs DNA-binding fluorescent dyes such as SYBR® Green fluorescent dye. Another method adds a target-specific oligonucleotide probe that is labeled at one end with a florescent tag and at the other end with a florescent quencher (FRET Probe). Fluorescence resonance energy transfer (FRET) is an energy transfer mechanism between two fluorescent molecules. In the TaqMan® variant, the fluorescent label at one end of the oligonucleotide is excited at its specific fluorescence excitation wavelength and this excited state is then nonradiatively transferred to the quencher molecule label at the other end of the oligonucleotide. In a quantitative PCR reaction, the fluorescent labels of those probes that bind to the DNA target are cleaved from the probe during primer extension releasing the fluorophore to emit signal at its specific fluorescence excitation wavelength without the energy being transferred. The signal emitted by the oligonucleotide FRET probe increases in direct proportion to the amount of PCR product in the reaction. By recording the amount of fluorescence emission at each cycle, the PCR reaction is monitored during the exponential phase where the first significant increase in the amount of PCR product correlates to the initial amount of target template. The higher the starting copy number of the nucleic acid target, the sooner a significant increase in fluorescence is observed. A significant increase in fluorescence above the baseline value measured during the 3-15 cycles indicates the detection of accumulated PCR product.

PCR is most useful in molecular diagnostic tests seeking presence or absence of a pathogen or other disease associated DNA sequence. Quantitative PCR is more advantageous when the diagnostic question includes the quantitative assessment of pathogen load.

To date, the U.S. Food and Drug Administration has cleared eight different PCR-based in vitro molecular diagnostic tests for diagnostic use in the United States. All of these tests are supplied as wet reagent sets that must be stored at -20°C. All of these tests include a set of oligonucleotides designed to amplify a target sequence associated with the disease of interest and an additional primer or primer pair designed as an internal control, which is amplified simultaneously with the target sequence of the disease of interest in one reaction tube. The internal control verifies successful DNA isolation and excludes false-negative results.

Quantitative PCR-based molecular diagnostic tests are commercially available from suppliers such as Qiagen Diagnostics (Qiagen Hamburg GmbH, Königstraße 4a 22767 Hamburg, Germany) which offers 60 different qPCR-based tests. As in the case of PCR-based molecular diagnostics, the commercial diagnostics sold by Qiagen Diagnostics are supplied as wet reagent sets that must be stored at -20° C and include an internal control.

Molecular diagnostic testing utilizing PCR or qPCR amplification of patient DNA samples includes multiple steps and requires highly trained laboratory personnel. A reaction mixture must be prepared in step-wise fashion and loaded into microtubes or into the wells of a multi-well plate, followed by each patient's DNA sample being loaded into a separate microtube or plate well. The reaction mixture includes oligonucleotide primers designed to amplify the target sequence, other oligonucleotides serving as internal control, DNA polymerase, dNTPs (dATP, dCTP, dGTP and dTTP), reaction buffer and magnesium chloride. In the case of PCR, the reaction mixture typically also includes a water-soluble dye. Several components of this reaction mixture, including DNA polymerase and dNTPs, must be stored at -20°C between kit uses and must be maintained on ice while being added to the reaction mixture in order to avoid degradation and loss of functionality. Oligonucleotide primers and probes are also stored cold and must be brought from cold storage to the clinical work bench.

In large clinical diagnostic laboratories where many patient samples are analyzed daily, a bulk reaction mixture is prepared in advance. The entire bulk mixture must then be brought from a freezer over to the clinical diagnostic work bench area, thawed, stored at the bench in an ice bucket, and loaded into each reaction microtube or well of a PCR plate before the patient DNA samples are loaded. This process frequently results in pipetting or other experimental errors leading to false negative responses as well as inducing carry-over contamination (see Kwok, S. et al,, Nature 339:237-238 (1989)) leading to false positive responses.

The process of preparing a PCR reaction mixture carries substantive risk of contamination, most often caused by DNA samples from previous assays being transported by aerosols, clothing, hands or equipment (McNerney, R. (1977), Kolmodin and Williams, (2000)). Current procedures to avoid contamination include the use of three separate rooms: One used only for storage and preparation of PCR reagents; a second used only for preparation of samples and positive or internal controls; and a third room where thermocycling and PCR product analysis are performed (McNerney, R. (1977), Kolmodin and Williams, (2000)). There is a need in the art for a kit that includes PCR reagents and controls in a closed, contamination free and pre-loaded reaction tube or multi-well plate that can be stored at the same PCR preparation bench where patient DNA samples are loaded.

It is well known that oligonucleotides degrade when stored at room temperature in an aqueous solution, and are more stable when dehydrated. This is due, in part, to the partial annealing of different primers to one another forming "primer dimmers" (Handyside 1990). Primer dimmers are formed readily at room temperature in a liquid state. After 30 minutes, they can significantly inhibit the specific PCR product, some time completely preventing the formation of the desired specific product and thereby generating false negative results (Chou 1992). Longer incubations (hours to days) results in complete lack of the PCR specific product (Bloch et al 1996).

Oligonucleotide primers and probes are, therefore, typically dehydrated for delivery and frozen for long-term storage. Enzymes, including DNA polymerase, when left at room temperature, deteriorate and loose functionality over time. In addition, dehydration of an enzyme causes a rapid decline in enzymatic activity. Water forms a protective wrapping around enzymes stabilizing their tertiary structure and blocking reactions with other reagents which can be found on the macromolecular surface. Drying an enzyme, in any manner, without providing a replacement aqueous wrapping instigates a loss of the enzyme's biological activity.

The identification of chemical additives that might be effective stabilizing specific enzymes for long term storage and utilization in laboratory processes has been a focus of scientific research. Various additives have shown positive stabilization effects for specific enzymes, but not for others. In some cases, a stabilizing agent has been shown to improve stabilization at room temperature for extended periods, but not to provide protection from the effects of dehydration. For example, while Ball et al. (1943) demonstrated that sucrose was effective in stabilizing certain enzymes in solution, Colaco et al (1992), found that sucrose was ineffective as a stabilizer for DNA polymerase.

Gelfand et al. (US 6,127,155) disclosed a method of increasing the stability of a DNA polymerase involving non-ionic polymeric detergents and Shultz (US 6,242,235) demonstrated similar increased stabilization of DNA polymerase in aqueous solutions containing polyethoxylated amine surfactants. However, both of these approaches require that the polymerase enzyme remain in a wet mixture solution. Accordingly, neither of these two approaches to stabilizing DNA polymerase would be effective for PCR reagent mixtures containing oligonucleotides where lyophilization or other drying is required for long-term storage at room temperature.

Clegg (1967), Mouradaian et al. (1984) and Roser (US4,891,319) identified trehalose as an agent that could be used to protect proteins and biological membranes from the deleterious effects of drying. Colaco and Roser (US 5,955,448), extended this finding to other non-reducing sugars, but only when an inhibitor of the Maillard reaction, such as an amino group, was added to the chemical mixture.

De Rosier et al. (US. 876,992 and US. 6,294, 365) present a method for preparing an enzyme that is both stabilized and lyophilized and Park et al. (US 5,861,251 and US. 6,153,412) describe preparation of a lyophilized reagent that includes basic components of the PCR reaction mixture other than the oligonucleotides. This process eliminates the need for DNA polymerase and dNTPs to be stored in the freezer and thawed prior to use and reduces some of the risk of cross contamination. Nevertheless, a diagnostic kit incorporating the lyophilized reagent described by Park et al., would still require that highly trained laboratory personnel cold store oligonucleotide primers and probes and add trace amounts of the oligonucleotides into each reaction microtube, retaining the risk of introducing experimental errors leading to false responses.

Rosado et al. (US 2003/0119042) describe a stabilized and dried PCR reaction mixture achieved by "a method consisting of bringing into contact, in one container, (a) an aqueous solution of a reaction mixture comprising at least one enzyme, and (b) an aqueous solution of a stabilized mixture comprising (i) at least one protective agent against drying, (ii) at least one inhibitor of the condensation reaction between carbonyl or carboxyl groups and amine or phosphate groups, and (iii) at least one inert polymer capable of generating a mesh structure preventing the mobility of the dried reagents." A review of the experimental data presented in the Spanish priority document of the Roasado et al. application, reveals evidence of deteriorating stability within a few weeks of a PCR reagent mixture prepared using the claimed three-component stabilization method. Thus, there is need in the art for an alternative methodology of providing ambient temperature stable kits for molecular diagnostics employing PCR or qPCR.

Klatser et al. (J. Clinical Microbiology, Vol 36, No. 6, 1798-1800, (1998)) describe a lyophilized PCR Mix into which trehalose was required to facilitate lyophilization, and into which they added a single pair of PCR primers prior to lyophilizing the reagent. Klatser et al. present data from two experiments, one in which the DNA polymerase used was AmpliTaq (Perkin-Elmer Cetus, Norwalk Conn.) and the other in which the DNA polymerase used was SuperTaq (HT Biotechnology, Cambridge, United Kingdom).

Klatser et al. note that the activity of their freeze-dried mixture was entirely lost after one week when the mixture included AmpliTaq and the mixture was not stored at 4° C or lower temperature. Klatser et al. surmise that the lack of extensive room temperature stability for the AmpliTaq mixture was due to the 50% glycerol solution in which AmpliTaq is supplied, as are most commercially available DNA polymerases. It was noted that the glycerol concentration increased during the lyophilization process as water disappeared. Since glycerol is hygroscopic, its presence in the final freeze-dried product likely results in a high moisture content, which may affect the stability of the product.

Klatser et al. found residual activity of their lyophilized mixture when rehydrated at three months when the DNA polymerase was SuperTaq, and Triton-X-100 was added to the distilled water used for rehydration prior to performance of the PCR reaction. Klatser et al. note that freeze drying of a mixture containing SuperTaq resulted in a dramatically lower glycerol concentration in the dry mixture (0.28% versus 0.48%) than found in the more common AmpliTaq solution. Klatser et al. could offer no explanation for this finding from their SuperTaq mixture experiment which limits the utility of their method for preparing diagnostic kits incorporating other commercially available DNA polymerases.

A limitation of the stabilized PCR reagents as described by Park et al. and Klatser et al. is that they require the use of a lyophilizing apparatus which is not an instrument commonly found in laboratories performing PCR. Thus, there would be a benefit to the art to have a method of preparing stabilized PCR reagents that can be performed utilizing inexpensive equipment that is common to PCR laboratories.

Moreover, to have utility, a molecular diagnostic kit must perform with a reliable consistent level of activity day after day. Current, state-of-the-art molecular diagnostic reagent sets are stored frozen between uses and are able to perform with the same level of activity the first day they are opened and months later. As we will demonstrate in an example, the use of lyophilization to prepare a room temperature stable reagent for PCR as described by Park et al. and Klatser et al. does not preserve consistency of activity level performance over time. Instead, analysis of results from PCR that utilized lyophilized reagents demonstrates a noticeable decrease in signal strength over time.

It is known that complete dehydration with lyophilization removes inter-molecular water molecules from enzymes, such as DNA polymerase. It is hypothesized that a DNA polymerase with inter-molecular water molecules completely removed by lyophilization, even in the presence of buffer and stabilizing agents, deteriorates in level of functioning over time more quickly than when not fully dehydrated or when dehydration is performed by methods other than lyophilization. Thus, there is still a need in the art for a method of achieving ambient stabilization of DNA polymerase in the context of a PCR reagent mix that does not involve lyophilization and retains sufficient reliability over time to provide trustworthy diagnostic results.

In addition, the prior art does not provide a method for providing an internal control in an ambient temperature stabilized kit for amplifying nucleic acid. Furthermore, the prior art does not provide a method for preparing an ambient temperature stabilized reagent mixture or kit that includes a fluorescent labeled oligonucleotide probe, as for example, is required to perform quantitative PCR.

It would be desirable, therefore, to provide an ambient temperature stabilized PCR reagent mix for amplifying nucleic acid that overcomes these and other disadvantages.

### SUMMARY OF THE INVENTION

One aspect of the invention provides a method for processing DNA polymerase and/or dNTPs for use in an amplification procedure, includes providing a solution mixture, the solution mixture including a DNA polymerase and/or dNTPs, a buffer solution and at least one stabilizing agent wherein said stabilizing agent comprises at least one sugar and at least one protein; and hydration reducing the solution mixture at a temperature between 25°C and 100°C.

Another aspect of the invention provides a kit for the amplification of a nucleic acid includes a hydration reduced solution according to the method of the invention including a thermophilic DNA polymerase and/or dNTPs, a buffer solution, at least one stabilizing agent, magnesium chloride, a set of two oligonucleotide primers, said oligonucleotide primers differing in sequence from each other, an oligonucleotide probe that differs in sequence from said set of two oligonucleotide primers, and a nucleic acid template.

Yet another aspect of the invention provides a kit for the amplification of a nucleic acid includes a first set of two oligonucleotide primers and one oligonucleotide probe, said oligonucleotide primers and probe differing in sequence from each other, and able to detect in a quantitative PCR reaction the presence of a unique nucleic acid sequence, and a second set of oligonucleotide primers and one oligonucleotide probe, said second set of oligonucleotide primers and probe differing in sequence from each other, and able to detect in a quantitative PCR reaction the presence of a distinct nucleic acid sequence that is different from the nucleic acid sequence being detected by the first set of primers and probe. The kit further includes a DNA polymerase enzyme, dNTPs (dATP, dCTP, dGTP and dTTP), a buffer solution containing one or more stabilizing agents and magnesium chloride. At least some of the reagents of the kit, including the DNA polymerase enzyme and the dNTPs, are hydration reduced by the method of the invention, and wherein the kit reagents together in a single mixture are capable of nucleic acid amplification activity after having been stored at ambient temperatures for up to 90 days and subsequently rehydrated.

The present invention is illustrated by the accompanying drawings of various embodiments and the detailed description and examples given below. The drawings and examples are for explanation and clarity. The detailed description and drawings are merely illustrative of the invention rather than limiting, the scope of the invention being defined by the appended claims. The foregoing aspects and other attendant advantages of the present invention will become more readily appreciated by the detailed description taken in conjunction with the accompanying drawings.

### BRIEF DESCRIPTION OF THE FIGURES

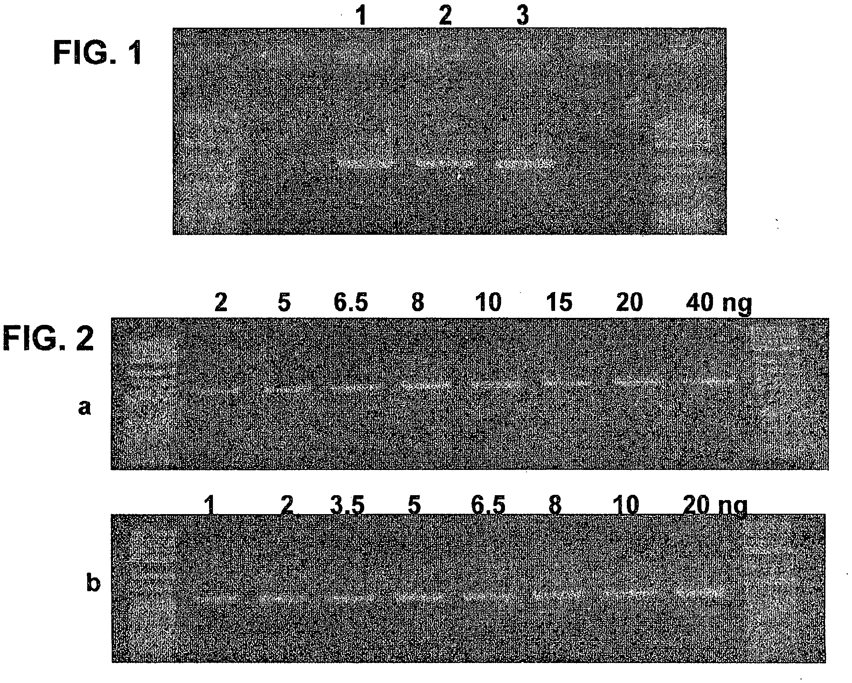

### Figure 1: PCR mix preparation and performance evaluation

The thermophilic DNA polymerase activity within the colored PCR Regular Mix was evaluated by the amplification efficiency of the APC amplicon. Lane 1: no treatment (wet mix), Lane 2: the mix was heated at 550C to reduce the hydration and re-hydrated prior to amplification, Lane 3: the mix was frozen at -200C, lyophilized over night and re-hydrated prior to amplification.

### Figure 2: PCR amplification using different template amount and DNA source

Mouse and human genomic DNA were efficiently amplified using the hydration reduced colored PCR Regular Mix of the invention. (a) IL1beta amplification of mouse genomic DNA using different template amounts (2-40ng) and (b) PLP amplification of human genomic DNA using different template amounts (1-20ng).

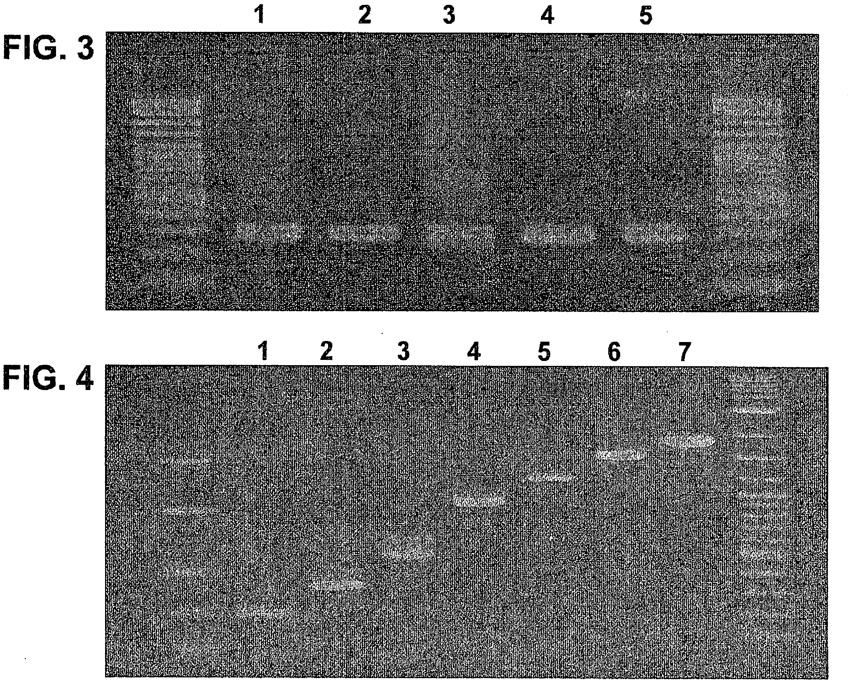

### Figure 3: Evaluation of different DNA polimerases performance included in the mixes and processed by the invention

ACTB amplification of representative samples using different brand DNA polymerases processed by the invention. Lanes: (1) Regular DNA polymerase within colored PCR Regular Mix- brand A; (2) Regular DNA polymerase within colored PCR Regular Mix- brand B; (3) Regular DNA polymerase within colored PCR Regular Mix- brand C; (4) Hot Start DNA polymerase within clear PCR Hot Start Mix-brand D; (5) Hot Start DNA polymerase within colored PCR Hot Start Mix- brand D.

### Figure 4: Evaluation of PCR amplification of random genomic sequences and amplicon sizes

Illustration of the ability of the PCR mixes of the invention to amplify different sequences and amplicon sizes. Lanes: (1) MAG, (2) BC12, (3) MHB, (4) p53, (5) IL1beta, (6) IL10 and (7) APC.

### Figure 5: PCR amplification evaluation of paraffin embedded DNA extracted samples using the PCR mixes of the invention

PCR amplification of (a) MAG, (b) BC12 and (c) ACTB genes using: (1) a Colored PCR Hot Start Mix stored at room temperature for 10 days, (2) a Clear PCR Hot Start Mix, (3) a Clear PCR Hot Start Mix amplifying a control human genomic DNA sample and (4) a Colored PCR Hot Start Mix stored at -200C.

### Figure 6: Dehydration kinetics and PCR amplification performance

PCR amplification of human genomic DNA for (a) CYP27, (b) PLP and (c) IL10 genes using colored PCR Regular Mix incubated at 550C for (1) 1hr, (2) 6hr and (3) 20hr.

### Figure 7: Shelf life estimation of the Colored PCR Regular Mix hydration reduced according to the invention and comparison to lyophilized samples

PLP amplification of samples: (a) lyophilized samples or (b) processed according to the invention. The different samples were incubated for 0, 1, 3, 6 and 8hr at 950C.

### Figure 8: PCR Hot Start Mix shelf life estimation tested using DNA samples extracted from paraffin embedded tissues

Representative samples showing the PCR amplification performance of PCR Hot Start Mix samples after incubation at 800C for: (1) 0hr, (2) 4hr, (3) 6hr and (4) 8hr on DNA samples extracted from: (a) mouse paraffin embedded tissue sections, (b) human paraffin embedded tissue sections and (c) human blood (genomic DNA used as control).

Top: BC1-2 PCR amplification product shows a 290bp band and bottom: ACTB shows a 300bp PCR product band.

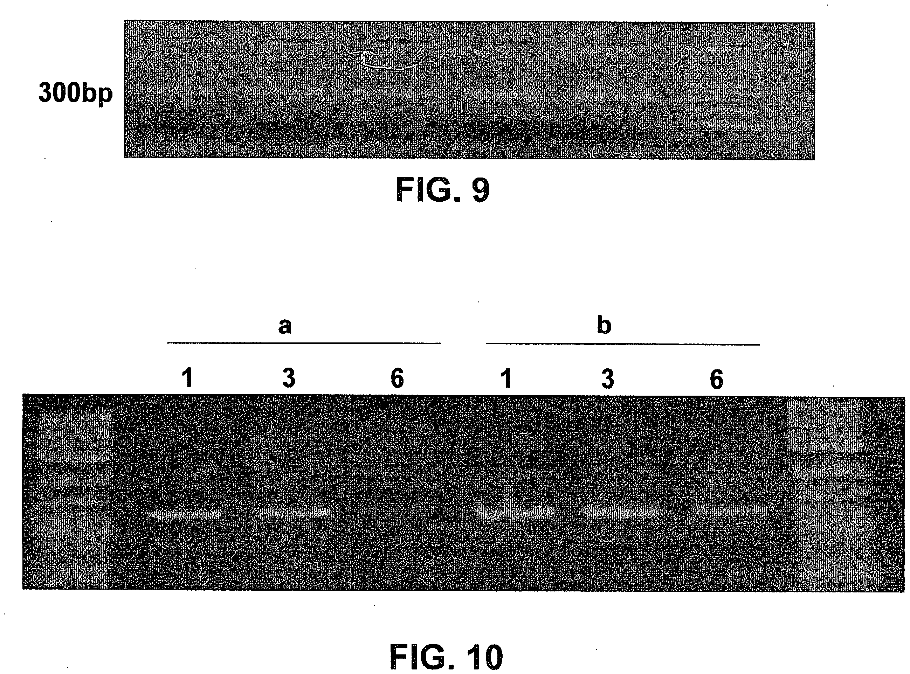

### Figure 9: Construction of a PCR mix internal control

Rehydration of hydration reduced PCR mix tubes including the positive control template and primers and PCR amplification. An expected band of about 300bp is clearly seen.

### Figure 10: Shelf life estimation of the Colored PCR Mix with incorporated primers hydration reduced according to the invention and comparison to lyophilized samples

PLP amplification of samples: (a) lyophilized samples or (b) processed according to the invention. The different samples were incubated for 1, 3 and 6hr at 950C.

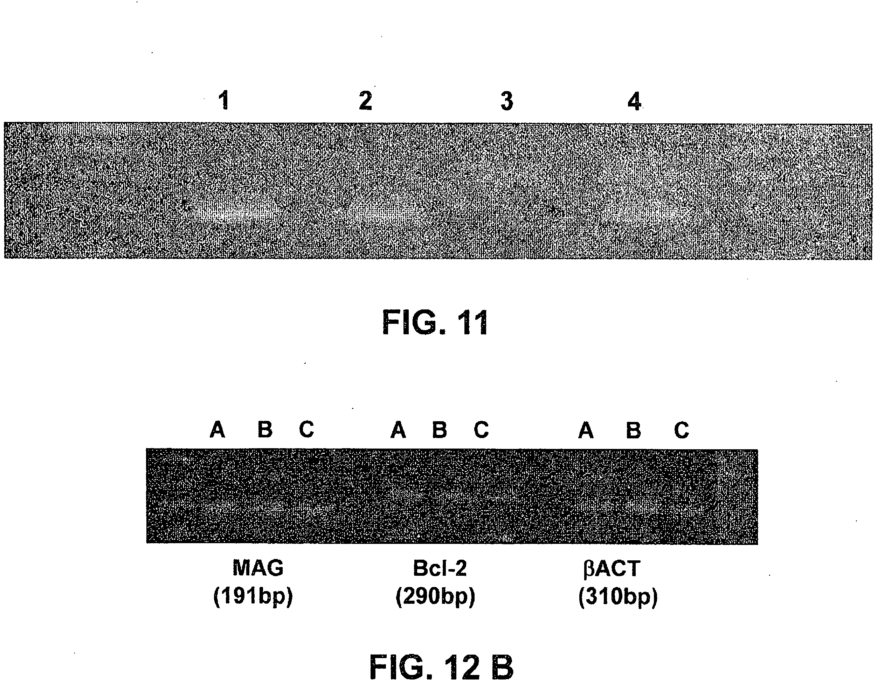

### Figure 11: Co-amplification of the internal PCR control and a target sequence

PCR amplification of: (1) hydration reduced PCR mix with additional MAG primers mix solution (wet primers), (2) hydration reduced PCR mix including the MAG primers, (3) hydration reduced PCR mix containing the positive control template and primers and (4) hydration reduced PCR mix including the MAG primers and the positive control template and primers.

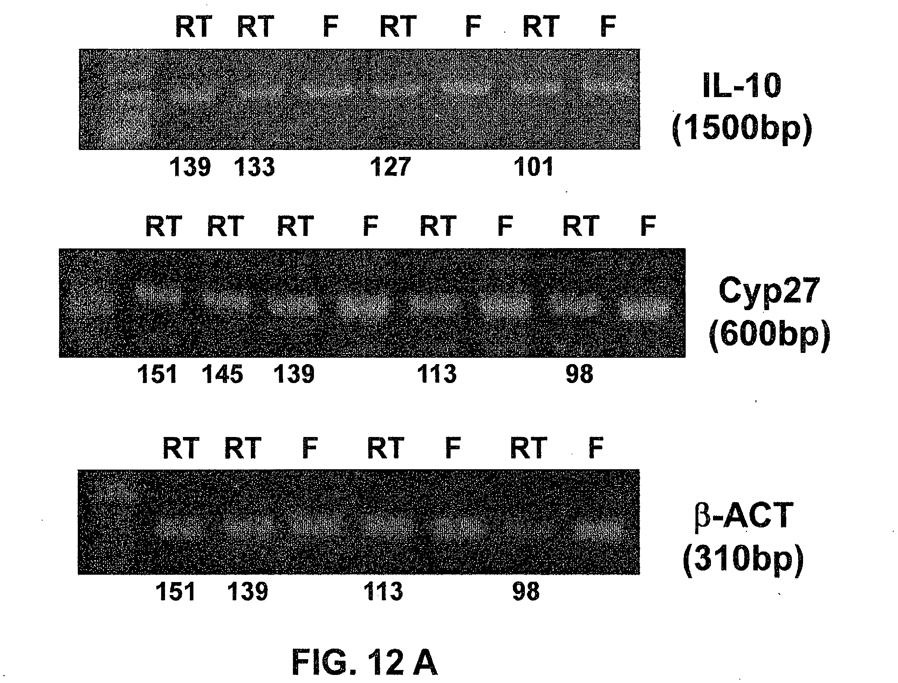

### Figure 12A: PCR Ready Mix Shelf life Experiment

Human genomic DNA (IL-10, Cyp27, β-ACT) was amplified using PCR ready mix stored at room temperature (RT) or frozen (F) from 98 to 151 days. IL-10 PCR amplification product shows a 1500bp band product band, Cyp27 shows a 600bp PCR product band and β-ACT shows a 310bp PCR product band.

### Figure 12B: PCR Ready Hot Start Shelf Life Experiment

Genomic DNA (MAG, Bcl-2, β-ACT) was amplified using PCR Ready Hot Start Supreme either (A) freshly prepared; (B) stored at -200C for 60 days or (C) stored at room temperature for 135 days. MAG PCR amplification product shows a 191bp band product band, Bcl-2 shows a 290bp PCR product band and β-ACT shows a 310bp PCR product band.

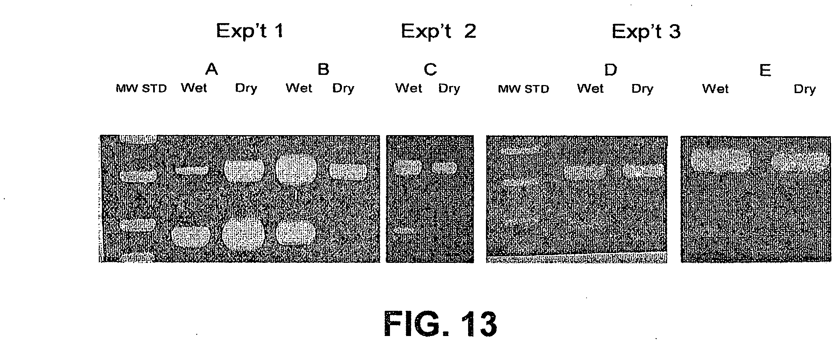

### FIG. 13 Requirement for adjusting the ratio balance of various reagent components to facilitate reaction optimization in a PCR duplex using a Regular PCR mix versus a stabilized PCR mix that will undergo hydration reduction

Conditions A-E describe the ratio of two sets of primers and DNA concentration as presented in Table 2 in Example 6. Exp't: Experiment, MW STD: Molecular weight standards.

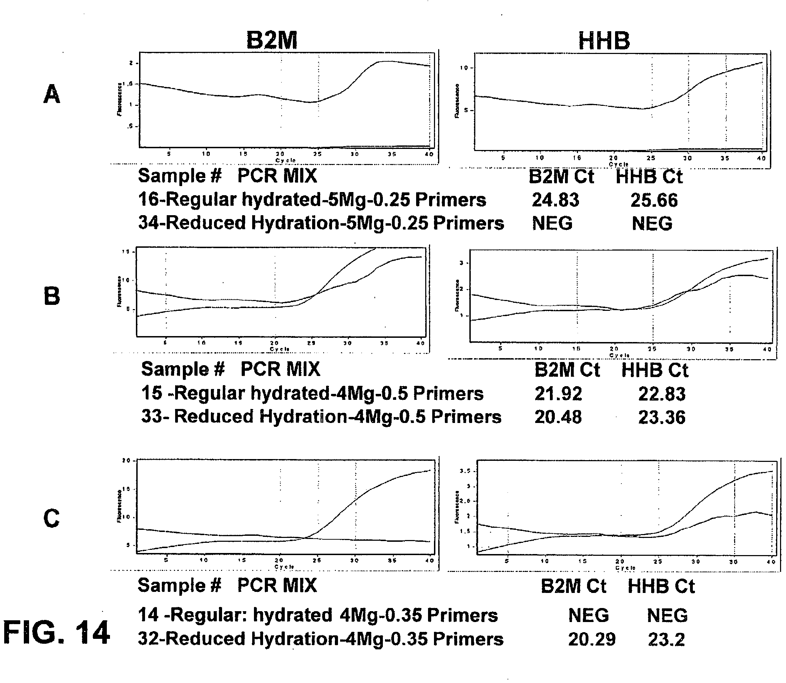

### Figure 14: Duplex Real Time PCR results of Regular QRT-PCR and stabilized QRT-PCR mix mixes

Comparison of duplex Real time PCR performance of Regular QRT-PCR mix (colored blue) and the stabilized QRT-PCR mix (colored red) with similar compositions. B2M- beta 2 microglobulin; HHB- human beta hemoglobin; Ct- cycle threshold; NEG- negative results according to the analysis parameters described in Example 7.

Improved results obtained with the Regular QRT-PCR mix under high magnesium concentration (5mM MgCl2 and 0.25µM Primers) (see 14-A). Similar performance of both mixes formats (4mM MgCl2 and 0.5 µM Primers) (see 14-B). Improved results obtained with the stabilized QRT-PCR mix using lower primer concentration (4mM MgCl2 and 0.35 µM Primers) (see 14-C).

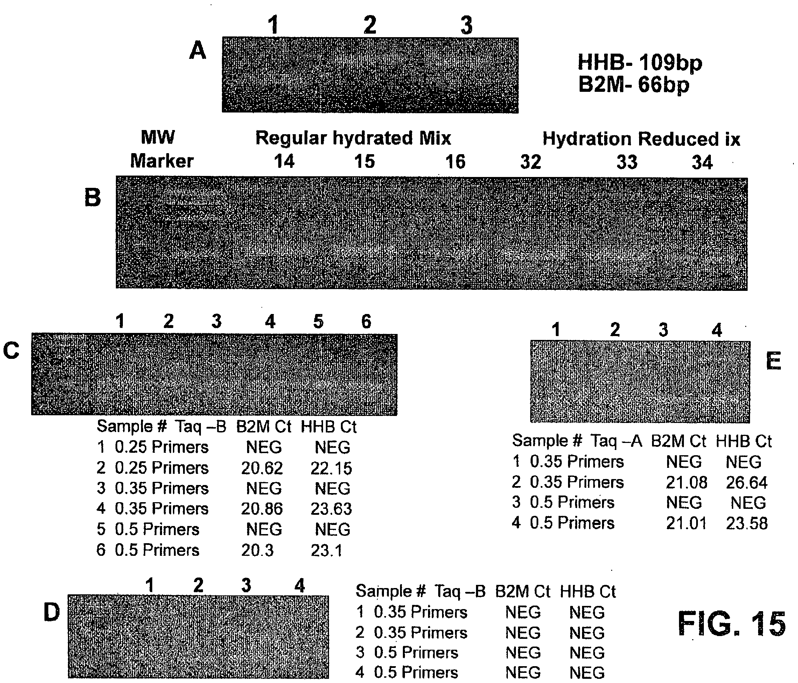

### Figure 15: Gel electrophoresis analysis of Duplex PCR amplification

Gel electrophoresis analysis of the duplex PCR amplification using different MgCl2 concentration, primers concentration and Hot Start Taq DNA polymerases from different suppliers (called Taq A & Taq B).

15-A (1) Single locus PCR amplification for B2M (66bp), (2) Single locus PCR amplification for HHB (109bp) and (3) simultaneous amplification of both loci with Hot Start Taq DNA polymerases from supplier A.

15-B Comparison of duplex Real time PCR amplification of a Regular QRT-PCR mix (samples # 14, 15, 16) and the stabilized QRT-PCR mix (sample # 32, 33, 34) with similar compositions using Hot Start Taq DNA polymerases from supplier A. (Samples from Fig. 14).

15-C Comparison of duplex Real time PCR amplification of a Regular QRT-PCR mix (samples # 1, 3, 5) and a stabilized QRT-PCR mix (sample # 2, 4, 6) with similar MgC12 concentration (4mM) and increasing primer concentration: (1-2) 0.25µM, (3-4) 0.35µM and (5-6) 0.5µM. Hot Start Taq DNA polymerases from supplier B.

15-D Comparison of duplex Real time PCR amplification of a Regular QRT-PCR mix (samples # 1, 3) and a stabilized QRT-PCR mix (sample # 2, 4) with similar MgCl2 concentration (5mM) and different primer concentration: (1-2) 0.35µM, (3-4) 0.5µM and Hot Start Taq DNA polymerases from supplier B.

15-E Comparison of duplex Real time PCR amplification of Regular QRT-PCR mix (samples # 1, 3) and a stabilized QRT-PCR mix (sample # 2, 4) with low MgCl2 concentration (2.5mM) and different primer concentration: (1-2) 0.35µM, (3-4) 0.5µM using the Hot Start Taq DNA polymerases from supplier A.

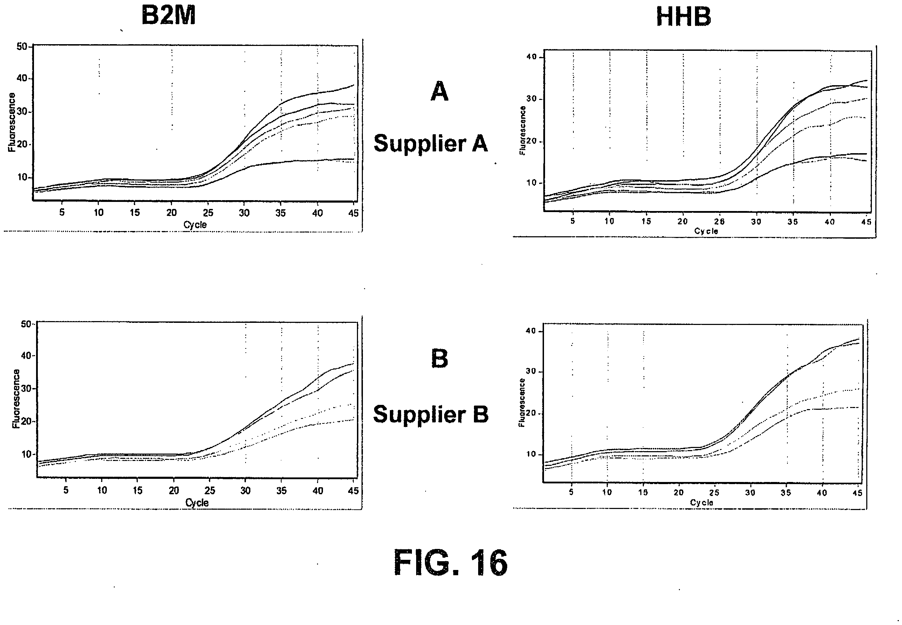

### Figure 16: Duplex Real Time PCR results of a stabilized QRT-PCR mix using different primer concentrations and Taq DNA polymerases

Optimization of duplex Real time PCR amplification for the stabilized QRT-PCR mix using (A) Hot Start Taq DNA polymerases from supplier A (AB gene) with 4mM MgCl2 concentration and increasing primer concentration 0.15µM, 0.25µM, 0.35µM and 0.5µM and (B) Hot Start Taq DNA polymerases from supplier B (ABI) with 5mM MgCl2 concentration and increasing primer concentration: 0.25µM, 0.35µM and 0.5µM.

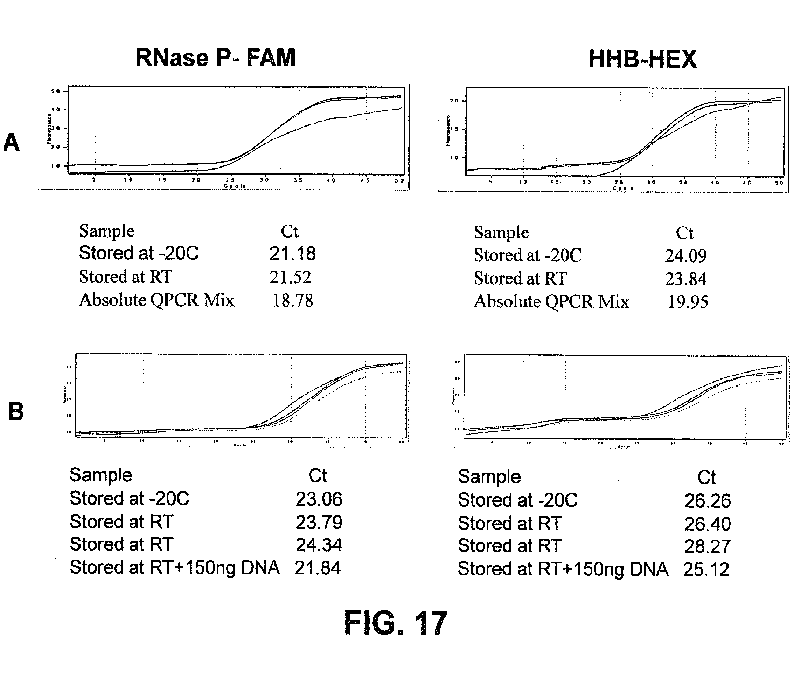

### Figure 17: Shelf life evaluation of a Real Time Duplex PCR using the Hydration Reduced QRT-PCR mix of the invention

Analysis of the reaction performance of a duplex QRT-PCR assay (RNase P & HHB) stored at room temperature (RT) in a regular hydrated commercial QRT-PCR mix and to the same assay pre-loaded into the stabilized Hydration Reduced QRT-PCR mix of the invention stored at -20°C for a period of 5 weeks (17-A) and 10 weeks (17-B).

### DETAILED DESCRIPTION

The present invention presents hydration reduced solutions and methods of making and using the hydration reduced solutions suitable for use in amplification procedures. The invention also describes kits having hydration reduced solutions that are room temperature stable and can be used for one step PCR reactions and quantitative PCR reactions routinely used in research and clinical diagnosis, such as a TaqMan, Molecular Beacon, Scorpion, Sunrise or Eclipse Probe assay. The hydration reduced solutions may also be used in all other comparable amplification and detections schemes using oligonucleotides. The rapid and simplified procedures enabled by the present invention can be performed by laboratory personnel with limited training and experience with reduced risk of carry-over cross contamination or experimental error. Furthermore, the kits of the present invention can be transported without a need for packing in dry-ice, enabling easier delivery, and substantial reduction in transport costs.

In one embodiment of the present invention, a hydration reduced solution includes at least one of a DNA polymerase and/or dNTPs (dATP, dCTP, dGTP and dTTP). In another embodiment, the hydration reduced solution includes both a DNA polymerase and dNTPs. The DNA polymerase may be a regular DNA polymerase, a thermophilic DNA polymerase, a recombinant DNA polymerase, a modified DNA polymerase or a hot start DNA polymerase.

The hydration reduced solution also includes a buffer compound and at least one stabilizing agent. In one embodiment, the stabilizing agent is a carbohydrate. In one embodiment, the carbohydrate is a non-reducing carbohydrate such as a non-reducing sugar. In one embodiment, the non-reducing sugar is sucrose.

Other non-reducing carbohydrates suitable for the present invention include, but are not limited to disaccharides, such as trehalose, trisaccharides and melezitose, non-reducing glycosides of polyhydroxy compounds selected from sugar alcohols and other straight chain polyalcohols, such as glycerol, glucitol, mannitol or galacitol. Other suitable carbohydrates include raffinose, stachyose, dextran.

In another embodiment, the carbohydrate is a reducing sugar. The reducing sugar may be for example, maltose, lactose, maltulose, iso-maltulose, lactulose, and combinations thereof.

J In another embodiment, the stabilizing agent is a protein. In one embodiment, the protein is bovine serum albumin (BSA). Other proteins suitable for the present invention include but are not limited to albumin from human serum (HSA) or avian sources and gelatin. In one embodiment, the BSA has a final concentration of between 0.1 to 5mg/ml BSA. In another embodiment the solution includes 1mg/ml BSA.

In one embodiment, the hydration reduced solution includes both a non-reducing sugar and a protein as the stabilizing agent. In one such embodiment, the stabilizing agent includes sucrose and BSA. In one embodiment, the sucrose concentration of the final solution is in the range of between 0.1 % and 20 % and the BSA concentration is 1 mg/ml. The concentrations of the sugar and the protein may be chosen depending on the application. In one embodiment, the sucrose concentration chosen depends on the type of DNA polymerase used in the solution. In an example, where the DNA polymerase is a regular DNA polymerase the sucrose concentration is in the range of 0.1% to 20%. In one embodiment where a regular DNA polymerase is used, the sucrose concentration is 3%. In another example, where the DNA polymerase is a hot start DNA polymerase, the sucrose concentration is in the range of 5% to 19%. In one embodiment where a hot start DNA polymerase is used, the sucrose concentration is 17 percent. Those with skill in the art will recognize that the concentration of the sugar depends on such factors as the type of sugar and the type of polymerase used in the solution.

The present invention provides a distinct, easier to implement and more reliable method of preparing ambient temperature stable PCR reagent mixtures than disclosed in prior art. In contrast to Rosado et al. (US. 203 / 0119042), the method of the present invention does not require a three-component stabilization including the use of an inert polymer capable of generating a mesh structure. In contrast to Colaco et al. (US 5,955,448), the method of the present invention does not require the addition of an amino group to inhibit the Maillard reaction. In contrast to De Rosier et al. (US 878,992 and US 6,294,365), Klatser et al. (1998) and Park et al. (US 5,861,251 and US 6,153,412), the method of the present invention does not require the use of a lyophilizing apparatus and provides solutions that preserve consistency of activity level performance over a sufficient duration of time as to be of practical use for diagnostic applications.

A further embodiment of the present invention includes additional reagents in the solutions. For example, a solution, as described above, containing DNA polymerase, dNTPs, a buffer compound containing one or more stabilizers may include other ingredients used in PCR amplification and may be processed in accordance with the method of the invention. A solution used to perform PCR may include magnesium chloride, water-soluble dye for direct tracking of PCR separation in an electrophoresis gel, target specific primers, and a second set of primers for performance of a duplex PCR amplification. Said solution could further include target DNA to serve as an internal control.

For a second example, a solution containing DNA polymerase, dNTPs, a buffer compound containing one or more stabilizers and other ingredients used in quantitative PCR amplification may be processed in accordance with the method of the invention. Such a solution used to perform quantitative PCR may include magnesium chloride, target specific primers, SYBR® Green and a reference dye. Alternatively within the second example, the solution may include magnesium chloride, target specific primers, a fluorescence resonance energy transfer (FRET) probe, a second set of primers and a second FRET probe for performance of a duplex PCR amplification. Said solution could further include target DNA to serve as an internal control.

Fluorescent labeled oligonucleotide probes and dual labeled fluorescence resonance energy transfer (FRET) probes are routinely dehydrated and later rehydrated prior to use without a noticeable loss of functionality because of the dehydration. However, in the known prior art, such fluorescent labeled probes have been dehydrated alone or in combination with other oligonucleotides. There is no teaching in the prior art to predict what effect would be had on the subsequent functionality of a fluorescent emitting agent, such as SYBR® Green, or an oligonucleotide with a detectable label moiety, such as a fluorescent labeled probe, that has been subjected to hydration reduction or dehydration, by any means, including heating or lyophilization, while in a solution that includes DNA polymerase, dNTPs, magnesium chloride, and a buffer containing stabilizing agents.

Accordingly, prior to the teachings of the present invention, it was not known to those skilled in the art that quantitative PCR analysis of DNA could be performed using a solution that contained a fluorescent emitting agent, such as SYBR® Green or an oligonucleotide with a detectable label moiety, such as a fluorescent labeled probe, together with DNA polymerase, dNTPs, magnesium chloride, and a buffer containing stabilizing agents, in the event that such a solution was hydration reduced or dehydrated, by any means including heating or lyophilization, and later rehydrated.

As will be illustrated in the examples provided below, solutions containing stabilizing agents having a sugar and a protein (for example sucrose and BSA) are able to be stored at ambient room temperature after the solutions have been "hydration reduced." As used herein, the term hydration reduced refers to the reduction of the water in the solution by at least 45 percent. More particularly, the reduction in water contained in the solution is between 50% and 80%. In other embodiments, the reduction of water is about 90 percent. In one embodiment, the solution is hydration reduced such that the percentage of remaining water is between 25 and 45 percent. In other embodiment, the reduction of water is between 80 and 99 percent. As those with skill in the art recognize, the removal of 100 percent of the water in solutions useful for performing PCR or other amplification procedures may decrease the efficacy of the solution. Thus, a hydration reduced solution having between 50 and 90 percent water reduction and that is room temperature stable is desirable.

Hydration reduction may be performed by any method known in the art where the temperature of the drying procedure is above 0°C and does not exceed 100°C. In one embodiment, the solution is hydration reduced at 55°C. In one embodiment, the solution is hydration reduced using an oven. In this embodiment, the solution is dried in an oven with a temperature between 25°C and 95°C. The length of time to achieve the desired amount of hydration reduction will depend on such factors as the drying temperature and the amount of solution to be hydration reduced. In one embodiment, the solution is dried at 35°C for about 12 hours. In another embodiment, the solution is dried at 80°C for 20 minutes.

The method of hydration reduction may include, but is not limited to, freeze drying, fluidized-bed drying, drum drying, drying at ambient temperature and atmosphere pressure, drying at ambient temperature and decreased pressure, drying at elevated temperatures and atmospheric pressure and drying at elevated temperatures and decreased pressure. As will be shown in Example 2, drying at elevated temperatures, as for example in an oven, confers better stability than lyophilization. In addition, oven drying is much simpler, technically, then any other drying method. It is therefore the preferred method of drying.

The hydration reduced solutions comprising DNA polymerase, and/or dNTPs, and also containing buffer compounds and stabilizing agents processed by the method of the invention can be stored at ambient room temperatures without reduction in DNA polymerase activity and without reduction in dNTPs activity for at least 90 days. The hydration reduced solutions may be stored at ambient room temperature for longer periods of time with minimal reduction in dNtps activity. In one example, the hydration reduced solutions may be stored at ambient room temperature for up to 24 months. The solutions of the invention may be used in any procedure utilizing DNA polymerase and/or dNTPs, such as procedures of amplification of nucleic acids such as PCR and qPCR. As used herein, with respect to storage or drying, ambient, or "room temperature" is generally about 20 degrees C.

Another embodiment of the invention provides kits containing hydration reduced solutions. The hydration reduced solutions of the present invention may be provided pre-loaded into a single reaction microtube, into a reaction microtube within a strip of reaction microtubes or into one or more well of a multi-well plate. Thus prepared, the reaction microtube, microtube strip, or multi-well plate can be utilized as a ready-to-use kit for DNA amplification by PCR or qPCR.

Another embodiment of the invention provides hydration reduced solutions containing more than one set of oligonucleotide primers capable of performing a duplex or multiplex amplification reaction. A further embodiment provides for the inclusion within hydration reduced solutions of an internal control assay or a positive control assay.

An internal control in a PCR reaction requires the inclusion within the reaction of a segment of target DNA. There is no known prior art demonstrating the effect on a dehydrated PCR or qPCR mixture that includes target DNA. As will be evident from the examples, it is a teaching of the present invention that one or more segments of target DNA can be added to a PCR reaction mixture that is then hydration reduced and later rehydrated without negatively affecting the amplification of the target nucleic acid.

In addition to the segment of target DNA, an internal control in a PCR reaction also requires the inclusion within the reaction of a second set of PCR primers. A second set of PCR primers is also required for a positive control. These duplex assays must then be capable of simultaneous amplification of more than one amplicon.

It is known that preparation of a duplex or multiplex PCR reaction requires special adjustments in the concentrations of various reagent components of the mixture so as to prevent one of the PCR primer sets from dominating the PCR reaction activity and preventing adequate amplification during the PCR reaction by the second set of primers. The different primer sets need to be balanced to achieve similar affinity for their respective templates. The enzyme processing should be similar for each amplicon analyzed, although some difference in size and sequence may exist. The quantity of MgCl2 often needs to be increased or decreased and the DNA polymerase activity stimulated or partially repressed to achieve an equivalent amplification of the different amplicons. Thus, optimal simultaneous amplification within a PCR assay mixture requires adjustments in the ratio balance of the DNA polymerase, MgCl2, quantity of each template targeted and each set of primers. These adjustments are determined empirically for each specific multiplex assay, depending upon the primer sequences, templates and polymerase. However, once determined, these adjustments form a condition set that can be used again and again when replicating the multiplex reaction.

Prior to the teachings of the present invention, it was not known to those skilled in the art that a multiplex PCR reaction mixture could be prepared with the requisite special adjustments in the case where the reaction mixture would be hydration reduced or dehydrated, by any means including lyophilization or heating, and later rehydrated, as is required for achieving an ambient temperature stabilized PCR or qPCR kit. In fact, as will be demonstrated in the examples, using the condition set formulated for a particular multiplex assay when applied to a traditional PCR reaction that does not undergo hydration reduction will typically fail to provide comparable simultaneous amplification of the same multiplex assay when replicated using a PCR mix that undergoes hydration reduction.

A teaching of the present invention is that during hydration reduction of a stabilized PCR amplification mixture, the components in the reaction mixture interact with each other in a different manner than they do in a PCR mixture that does not undergo hydration reduction. Furthermore, when a PCR reaction mixture is significantly hydration reduced or fully dehydrated, whether by lyophilization or other means, and then later rehydrated just prior to initializing a PCR reaction, the local concentration of reagents within the mixture is likely different for a period of time after rehydration than it was prior to the original dehydration.

The condition set reflecting adjustments in the concentrations of various reagent components of a multiplex reaction mixture that would be made by following methods in the current art using a traditional non-stabilized PCR reaction mixtures that does not undergo hydration reduction, tends not to provide the correct local concentrations of reagents within the mixture to perform a multiplex reaction when the mixture is dehydrated and later rehydrated in order to initialize a PCR reaction. Attempting to replicate known duplex reaction conditions that were based on a PCR mix that did not undergo hydration reduction would lead to the false conclusion that a simultaneous amplification multiplex reaction cannot be performed using a hydration reduced PCR mix.

In contrast to such a false conclusion, a fundamental teaching of this invention, that will be apparent from the examples, is that a Hydration Reduced PCR mix can be optimized to perform multiplex QRT-PCR reactions, but the calibration requires abandonment of the balance of the components concentrations derived from experimentation for the particular multiplex assay application using a Regular QRT-PCR mix and empirical assessment of said balance of component concentrations using a Hydration Reduced QRT-PCR mix.

Another embodiment of the present invention is the preparation of hydration reduced solutions for the performance of duplex and multiplex quantitative real-time PCR (QRT-PCR). In a typical QRT-PCR amplification reaction, an additional molecule is added to the reaction, a dual labeled probe with a fluorescent dye reporter and a quencher. This extra molecule, with different physical characteristic than a regular oligonucleotide, may function differently upon combination with the reaction stabilizers used in the mix and the hydration reduction process. This might profoundly affect its interaction with the DNA template during the PCR amplification and hybridization, repercussing in the reaction performance. As will be evident from the examples, the condition set balance of the ratio of the components of a duplex or multiplex assay in a Regular QRT-PCR mix that is not going to be hydration reduced, will not properly perform in the hydrated reduced format, and a recalibration of the balance in the ratio of the mix components is required to enable simultaneous amplification of the amplicons. Therefore, in contrast to the expectation of those skilled in the art, it is the teaching of this invention that a Hydration Reduced QRT-PCR mix can be optimized to perform multiplex QRT-PCR reactions, but differently from experimentation with a Regular QRT-PCR mix and recalculation of said balance of component concentrations using a Hydration Reduced QRT-PCR mix should be performed empirically.

The examples will show that certain components are better raised or lowered when recalibrating from the condition set of a regular QRT-PCR mix to a mix that will be hydration reduced, depending upon the specific assay, polymerase and template DNA targets. Specifically, the relative concentration of the various primer-probe sets, of MgCl2, and of template DNA may each prove optimal at higher or lower levels for different multiplex assays. With regard to the concentration of DNA polymerase, the finding is more indicative of a pattern. It is a teaching of the present invention that PCR and QRT-PCR mixes that undergo hydration reduction provide more robust reactions than comparable regular PCR and QRT-PCR mixes and, therefore, tend to achieve amplification under more extreme conditions and may require less enzyme.

As will be evident from the following examples, the solutions of the invention can be stored without ice (i.e. at ambient room temperature) at the same PCR preparation bench where patient DNA samples are loaded. Preparation for the PCR or quantitative PCR can thus be carried out in a single step of adding a diluted DNA sample into the hydration reduced solution of the invention to start the PCR or qPCR reaction. For example, 15-100ng of template DNA diluted in PCR grade water may be added to the hydration reduced solution of the invention to form a 25 µl mixture. The hydration reduced solution of the invention does not need to be completely dissolved prior to starting a PCR or qPCR reaction, but may be partially dissolved, as for example, by vortexing for 2-4 seconds.

### Examples

The following examples illustrate the room temperature stable PCR ready mix compositions and some applications in DNA analysis.

### Materials & Methods

PCR Regular Mix- Enzyme buffer x 1 (10mM Tris pH 8.3, 40mM KCl), 1.5mM MgCl2, 0.2mM dNTPs mix, 0.3µM primer mix and 0.5 units thermophilic DNA polymerase

Colored hydration reduced PCR Regular Mix- Enzyme buffer x1 (10mM Tris pH 8.3, 40mM KCl), 1.5mM MgCl2, 0.2mM dNTPs mix, 0.3µM primer mix, 0.5-6.4 units thermophilic DNA polymerase, 3% sucrose, 1mg/ml BSA and 0.04% Cresol red.

Clear hydration reduced PCR Regular Mix- Enzyme buffer x 1 (10mM Tris pH 8.3, 40mM KCl), 1.5mM MgCl2, 0.2mM dNTPs mix, 0.3µM primer mix, 0.5-6.4 units thermophilic DNA polymerase, 3% sucrose and 1mg/ml BSA.

PCR Hot Start Mix- Enzyme buffer x1 (10mM Tris pH 8.3, 50mM KCl), 2mM MgCl2, 0.2mM dNTPs mix, 0.3µM primer mix and 1-2.5 units Hot Start thermophilic DNA polymerase.

Colored hydration reduced PCR Hot Start Mix- Enzyme buffer x 1 (10mM Tris pH 8.3, 50mM KCl), 2.5mM MgCl2, 0.2mM dNTPs mix, 0.3µM primer mix, 1-2.5 units Hot Start thermophilic DNA polymerase , 17% sucrose, 1mg/ml BSA and 0.07% Orange G.

Clear hydration reduced PCR Hot Start Mix- Enzyme buffer x1 (10mM Tris pH 8.3, 50mM KCl), 2.5mM MgCl2, 0.2mM dNTPs mix, 0.3 µM primer mix, 1-2.5 units Hot Start thermophilic DNA polymerase, 17% sucrose and 1mg/ml BSA.

Exemplary preparation of hydration-reduced ready to use PCR Mix. As an example, a red color mixture with 0.8 units thermophilic DNA polymerase is described.

Preparation of reaction mixture for a PCR reaction with a rehydrated volume of 25 µl:

1. Add a volume of PCR stock solution, concentrated. The solution contains, after rehydration, the following components: 10mM Tris pH 8.3, 40mM KCl, 1.5mM MgCl2, 3% sucrose, 1mg/ml BSA and 0.04% Cresol red.

2. Add 0.2mM dNTPs mix.

3. Add 0.8-units thermophilic DNA polymerase, from a 5 units/µl enzyme stock.

4. Add a primer mix to achieve a concentration of 0.3 µM.

5. Mix the components and dispense into a 0.2 ml PCR tube.

6. Perform hydration reduction by drying in an oven at 55oC for 90 minutes.

7. Cool tube at room temperature, and close the tube cap.

8. Store at room temperature up to 24 months.

9. Rehydrate before use.

In this example, to perform the PCR reaction, the hydration reduced solution is rehydrated with water and DNA, and then run in a PCR machine. The drying volume of the reaction mixture in different preparations can be substantially different then in the above example, for the following reasons:

The reaction mixture may contain additional primers and/or probes (or none at all), or more units of enzyme are being used, or the volume of the PCR reaction is smaller or higher (for example from 5- 100 µl), or the reaction use a Hot Start mix which has a higher sucrose content and therefore higher volume. Different volumes to be dried require different drying times at 55oC. In practice, drying at 55oC for 1-3 hours is sufficient for all preparations.

### Example 1

### PCR mixes performance

In a first experiment, the thermophilic DNA polymerase activity within the colored PCR Regular Mix was evaluated. The colored PCR regular mix was amplified: (1) with no further treatment (wet mix), (2) was heated at 550C to reduce hydration and re-hydrated prior to amplification, or (3) was frozen at -200C, lyophilized over night and re-hydrated prior to amplification.

Sixty nanograms of genomic DNA were amplified to obtain an 1800bp PCR product (APC gene, primers SEQ ID 21 & 22) according to the following protocol: 3 min at 950C, followed by 35 cycles of 30 sec at 950C; 60 sec at 590C, 2 min at 720C and a final step of 10 min at 720C. PCR amplified products were separated in a 1.5% agarose gel and stained with ethidium bromide.

As seen in Figure 1, similar activity could be detected in the three samples. Therefore it can be concluded that the mix composition in which the drying process of the invention is taking place protects the enzyme from activity deterioration, or at the most it exerts the same impact as the one observed for the standard/classic lyophilization method commonly used to preserve protein and/or enzyme activities.

In a following experiment, at least 9 different thermophilic DNA polymerase enzymes were tested for their amplification performance after being processed as described in the method of the invention. All the tested enzymes showed high performance when tested for numerous amplicons.

Although in the majority of the tests, 20-75ng of genomic DNA were amplified using the PCR protocol described above, other different DNA sources and DNA template amount (0.1-200ng genomic DNA) were also tested and efficiently amplified (as seen in Figure 2).

Figure 3 exemplifies the PCR amplification performance for the ACTB gene of three different thermophilic DNA polymerases and a Hot Start thermophilic DNA polymerase enzyme mixed within a colored or a clear PCR mix described above.

Figure 4 illustrates the ability of using the PCR mix of the invention to amplify different sequences and amplicon sizes as represented by these 7 exemplars (mouse and human DNA). More than 24 different gene or genetic marker sequences were tested and successfully amplified. Some of the amplified genes, genetic markers and their primers are listed in Table 1.

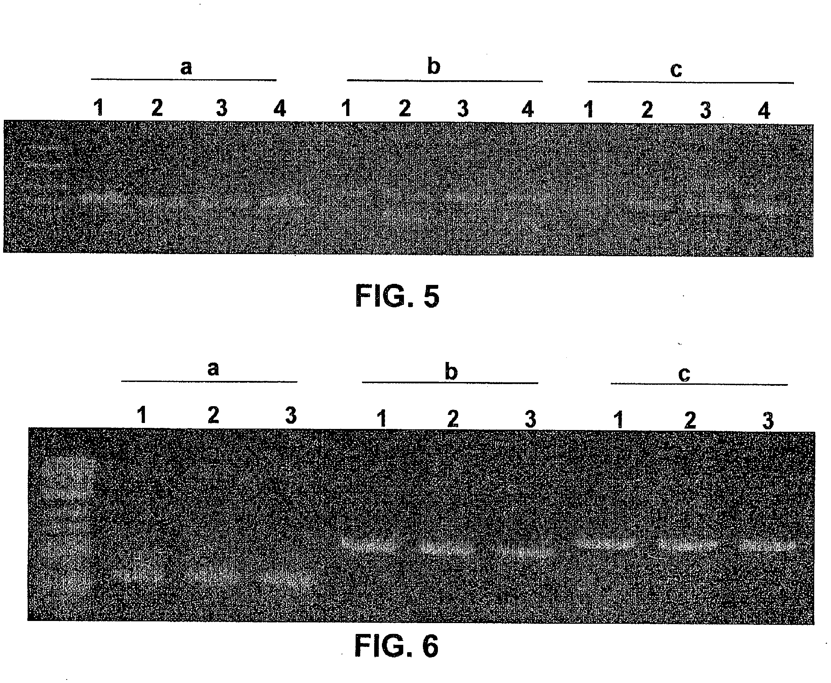

Figure 5 shows the ability of the Hot Start PCR mix to amplify even poor quality DNA, as for example DNA extracted from formalin fixed paraffin embedded tissues. The mix of the invention overcomes the difficulty of small amount and poor quality DNA template.

Five microliter of DNA samples that were extracted from paraffin embedded sections, were tested for MAG, BC1-2 and ACTB (Table 1) amplification using: (a) a Colored hydration reduced PCR Hot Start Mix stored at room temperature for 10 days, (b) a Colored hydration reduced PCR Hot Start Mix stored at -200C, or (c) a Clear hydration reduced PCR Hot Start Mix which was also used to compared its efficiency in amplifying human genomic DNA (25ng) extracted from blood and the following PCR protocol: 10 min at 950C, 45 cycles of 30sec at 950C, 30sec at 550C, 30sec at 720C and a final step of 10min at 950C. PCR products were visualized on a 2% agarose gel after staining with ethidium bromide.

Both mixes used, colored and clear, stored at -200C or room temperature, render a PCR product with similar efficiency, as reflected by the bands intensity on the gel (Figure 5).

The Colored hydration reduced PCR Hot Start Mix is useful when results are analyzed as positive or negative for PCR amplification. Utilization of "ready to use PCR mixes" eases the analysis of samples and avoids confusion and contamination. The Clear hydration reduced PCR Hot Start Mix is recommended when other molecular biology techniques (rather than a simple electrophoresis gel) are used to analyze the DNA sample and the presence of a dye may interfere with results.

In a variety of experiments, different sources and qualities of template DNA were studied. As for human source, genomic DNA extracted from peripheral blood lymphocytes, buccal swab, hair bulb, and histology slides, etc. were successfully amplified. Regarding mouse and rat, genomic DNA extracted from peripheral blood lymphocytes, different organs, tail (usually a bad quality DNA as result of the many impurities present) and paraffin embedded tissue sections were examined. Additional test were performed with viral, plant, insect, bacteria and yeast DNA samples.

The hydration reduced PCR mixes of the invention showed high PCR amplification performance regarding different DNA sequences, as demonstrated by the large number of amplicons tested, as well as regarding the DNA template quality which is demonstrated by the used of highly degraded DNA (usually obtained from paraffin embedded tissue sections) as well as regarding the DNA extract impurities (usually present in the DNA obtained from mouse tails) that eventually may inhibit the PCR amplification.

In order to determine if different durations of hydration reduction will affect a significant drop in enzyme activity or PCR amplification, three different hydration reduction regiments were applied to the PCR mixes of the invention. For example, when the Colored PCR Regular Mix was tested, sample tubes were heated for 1, 6 or 20 hours at 550C. After rehydration, the different samples were subjected to PCR amplification for three different amplicons using 25ng of human genomic DNA and the following amplification protocol: 3 min at 950C, followed by 35 cycles of 30 sec at 950C; 60 sec at 590C, 2 min at 720C and a final step of 10 min at 720C. PCR amplified products were separated in a 1.2% agarose gel and stained with ethidium bromide. The enzyme activity performance was evaluated by the comparison of the PCR product band intensity of the three CYP, PLP and IL-10 different amplicons.

It can be pointed that no significant difference, if at all, can be observed among the samples heated for the different time periods for any of the amplicons tested. Figure 6 illustrates the results of such an experiment.

### Example 2

### Shelf life extension of the PCR mixes of the invention

To estimate the shelf life of the PCR mixes of the invention, separate tests were performed for the regular thermophilic DNA polymerases and the Hot Start enzyme containing mixes.

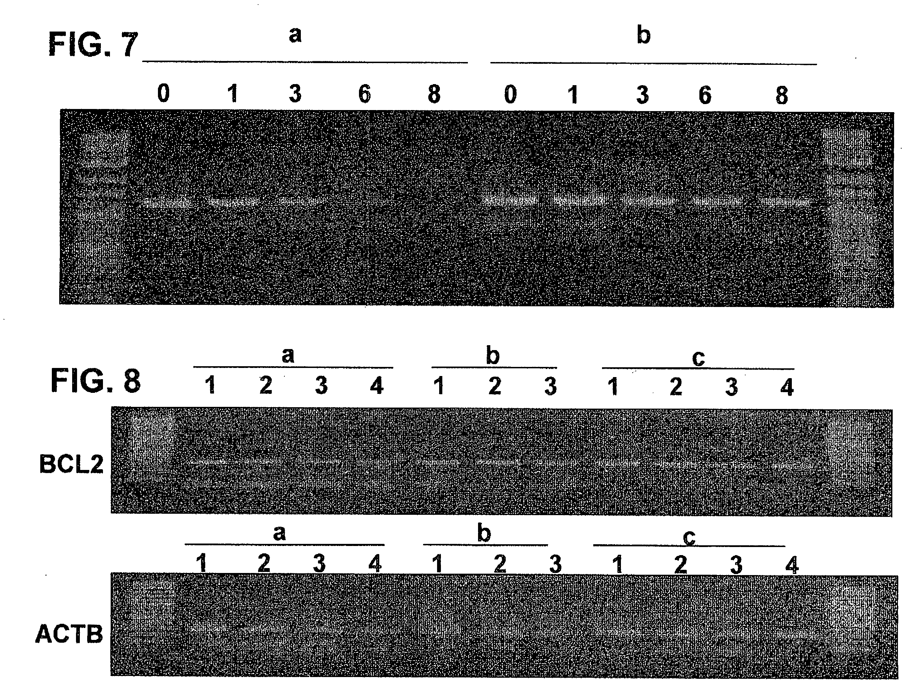

In an accelerated shelf life test for the stabilized Hydration Reduced PCR mix containing regular DNA polymerase, the mix containing tubes were incubated for 0, 1, 3, 6 and 8hrs at 950C and tested for PCR amplification efficiency of the PLP gene (Table 1). Human genomic DNA (25ng) was amplified using the following PCR protocol: 3 min at 950C, 35 cycles of 30 sec at 950C; 60sec at 590C, 2 min at 720C, and a final cycle of 10 min at 720C. Although a decline in the enzyme activity can be perceived (Figure 7b), the enzyme exhibited strong performance even after 8 hrs incubation at such high temperature.

Based on the Ahrenius accelerated shelf life test (ASLT) model and previous experiments in which the Q10 value was determined, we estimated the shelf life of the tested colored hydration reduced Regular PCR Mix to be equivalent to about 732 days at room temperature (RT) or 24 months.

In parallel, an accelerated shelf life test was performed for the same colored Regular PCR mix, but in this instance the dehydration process was performed by the commonly used lyophilization procedure, instead of the process used in the invention. The lyophilized tubes were incubated for 0, 1, 3 and 6 hours at 950C and tested for PCR amplification of the PLP gene (Table 1) with 25ng of human genomic DNA as described above. As seen in Figure 7a, the enzyme activity was significantly reduced just after 3 hr incubation.

In the last paragraph of Example 1, the experiment described a procedure to prepare the mixes of the invention for which the final dehydration state will be equivalent to the one achieved by the lyophilization process. Although the predicted enzyme stability would have been expected to be similar for both dehydrating procedures, a significant decline in the enzyme performance was evident.

The difference in the shelf life behavior for the examined PCR mixes dehydration procedures clearly demonstrates that the PCR mix preparation method used in the invention allows a better interaction between the DNA polymerase enzyme, the buffer constituents and the stabilizers, providing a stronger stability to the enzyme. Such increased stability is reflected by the prolonged period that the enzyme activity is preserved. According to the experimental results, the expected shelf life of the mixes of the invention is at least 2.5 times longer than the one expected for the lyophilized PCR mixes.

In the accelerated shelf life test for hydration reduced PCR Hot Start Mix, Colored PCR Hot Start Mix containing tubes were incubated for 0, 4, 6 and 8hrs at 800C and tested for PCR amplification for BC1-2 and ACTB genes (Table 1). DNA samples extracted from mouse and human paraffin embedded tissue sections were compared to 25ng of human genomic control DNA (extracted from blood) for amplification efficiency (Figure 8). The following PCR protocol was used: 10 min at 950C, 45 cycles of 30sec at 950C, 30sec at 550C, 30sec at 720C and a final step of 10min at 950C. PCR products were visualized on a 2% agarose gel after staining with ethidium bromide. Although a decline in the enzyme activity can be perceived, the enzyme exhibited strong performance even after 8 hrs incubation.

Based on the Ahrenius accelerated shelf life test (ASLT) model and previous experiments in which the Q 10 value was determined, we estimated the shelf life of the tested Colored hydration reduced PCR Hot Start Mix to be equivalent to 140 days at room temperature (RT), about 4.5 months.

### Example 3

### PCR Ready mixes with incorporated primers and internal control

A PCR assay mixture containing a positive control typically includes one set of oligonucleotide primers that are directed to a specific genetic region that is unique to the target DNA and a second set of oligonucleotide primers directed to a different genetic region that is common to a broader family of DNA. An internal control includes the elements of the above assay plus a sample of the DNA that contains the genetic region of the control primers and does not contain the unique genetic region of the target DNA.

In order to design an internal control for the PCR reaction, a synthetic DNA segment comprising the sequences of the ACTB primers (SEQ ID 7 & 8) was prepared and purified. This synthetic segment, also referred as the positive control template, was combined together with a ACTB forward and reverse primer mix and added to the PCR mixes of the invention prior to the hydration reduction process. Tubes containing the PCR mix and positive control template and primers were rehydrated and amplified using the amplification protocol: 3 min at 950C, 35 cycles of 30 sec at 950C; 60sec at 590C, 2 min at 720C, and a final cycle of 10 min at 720C. As seen in Figure 9, a clear band of about 300bp was obtained.

In other separated experiments, forward and reverse primer mixes were added to the wet PCR mixes of the invention and processed as previously described. All the PCR Ready mixes with incorporated primers rendered specific PCR bands after rehydration. These results demonstrate that the process of the invention is suitable for the preparation of sequence-specific-PCR mixes that are stable at room temperature and incorporate positive control or an internal control.

To estimate the shelf life of the above described sequence-specific-PCR mixes, an accelerated shelf life test was performed. Tubes containing the hydration reduced PCR mix and primers were incubated for 1, 3 and 6 hrs at 950C and tested for PCR amplification efficiency of the PLP gene (Table 1). Human genomic DNA (25ng) was amplified using the following PCR protocol: 3 min at 950C, 35 cycles of 30 sec at 950C; 60sec at 590C, 2 min at 720C, and a final cycle of 10 min at 720C. Similarly, an accelerated shelf life test was performed for the same PCR mix which was lyophilized instead of hydration reduced by the process described in the invention. As previously seen in example 2, the PCR efficiency (reflected by the PCR product band) was significantly reduced after 3 hr incubation for the lyophilized product, while considerable bands were observed for the mix processed according to the invention, even after 6 hr incubation at 950C (Figure 10).

Based on the Ahrenius accelerated shelf life test (ASLT) model, we estimated the shelf life of the tested sequence-specific PCR Mix to be equivalent to at least 550 days at room temperature (RT) or 18 months, twice the time of the lyophilized similar product.

In a following experiment, a combination of the previously mentioned compositions was tested: the PCR mix of the invention was hydration reduced in the presence of a sequence-specific set of primers (MAG) and a positive control synthetic template and a second primer set (ACTB). The resulting sequence-specific PCR Mix with the reaction internal control incorporated was tested for PCR amplification and compare to the hydration reduced PCR mix containing only one of the additional components at the time. Figure 11 illustrates the success of the co-amplification of a target sequence and the PCR reaction positive control.

It can be summarized, that the combined product described above, could be used for the simultaneous detection of different target sequences, and said products are stable at room temperature for at least 18 months.

**Table 1**

| **Loci** | **SEQ ID #** | **Primer Sequence** | **Product Size (bp)** |
|---|---|---|---|
| DIS199 | SEQ ID 1 | GGTGACAGAGTCAGACCCTG | |
| | SEQ ID 2 | CAAAGACCATGTGCTCCGTA | 100 |
| MAG | SEQ ID 3 | TCCACACAGAGCAACCCGGAC | |
| | SEQ ID 4 | ACACTCCACAGACAGGTTGAAG | 190 |
| BCl-2 | SEQ ID 5 | TCCTGTGCTGCTATCCTGCCA | |
| | SEQ ID 6 | GAGCAAGTGCAGCCACAATACT | 290 |
| ACTB | SEQ ID 7 | GTCCACCCACACTGTGCCCAT | |
| | SEQ ID 8 | GAACCGCTCGTTGCCAATAGT | 300 |
| MHB | SEQ ID 9 | CCAATCTGCTCACACAGGATAGAGAGGGCAGG | |
| | SEQ ID 10 | CCTTGAGGCTGTCCAAGTGATTCAGGCCATCG | 500 |
| CYP27 | SEQ ID 11 | AACCAGGACAATGCGGGCCAC | |
| | SEQ ID 12 | CTCTACCCTGTGGTCCCCACA | 580 |
| P53 | SEQ ID 13 | GCATTCTGGGACAGCCAAGTC | |
| | SEQ ID 14 | GTCATGTGCTGTGACTGCTTG | 900 |
| IL 1 beta | SEQ ID 15 | GGGCTGGAAAAATGGTC | |
| | SEQ ID 16 | TCTGGGGTTGATGTAGGA | 1180 |
| PLP | SEQ ID 17 | GATAACAGCTACCATGACAA CC | |
| | SEQ ID 18 | ATTCACTCAAAGGACACGAT GT | 1280 |
| IL-10 | SEQ ID 19 | GGGTTACTTGGGTTGCCAAGCC | |
| | SEQ ID 20 | TCTGTTTCCTATGTCACTCTCC | 1510 |
| APC | SEQ ID 21 | CAATAGTCAGTAATGCATGTGG | |
| | SEQ ID 22 | TTAGCAGAATCTGCTTCCTGTG | 1880 |

### Example 4

### PCR Ready mixes incorporating fluorescent labeled oligonucleotides

Using Real-Time quantitative PCR enhances the detection sensitivity as compared to regular PCR amplification to the degree that very small target DNA amounts can be detected: about 10pg in gene expression assays and 0.01 pg in contamination detection assays. An ambient temperature stabilized kit for performing a quantitative PCR reaction would incorporate in a single mixture, prior to that mixture being hydration reduced, all the components of the Clear hydration reduced PCR Hot Start Mix of the present invention, together with oligonucleotide primers, as demonstrated in Example 3 above, and a fluorescent labeled oligonucleotide or a fluorescent emitting agent such as SYBR® Green.

In order to determine if hydration reduction of a mixture containing a fluorescent labeled oligonucleotide together with the mixes of the invention will negatively affect subsequent PCR amplification and/or negatively affect detection of the fluorescent signal, D1S999 forward and reverse primers were prepared and purified, with a 6-FAM fluorescent label added to the 5'-end of the forward primer. These primers were added to the Colored hydration reduced PCR Regular Mix and to the Clear Hot Start PCR mix of the invention prior to the hydration reduction process. Tubes containing the PCR mixes and primers were rehydrated and amplified using the amplification protocols as described above. PCR products were visualized on a 2% agarose gel after staining with ethidium bromide and a band indicative of the predicted PCR product was observed. The PCR product was then analyzed using a fluorescent reader and signal was detected.

Thus, the findings of Examples 3 and 4 teach that the methods of the invention can be used to create a mixture containing all of the components required for performance of a quantitative PCR reaction, including unlabeled primers and a fluorescent emitting agent, such as a labeled primer, as well as a second set of oligonucleotides for a positive control, and a segment of target DNA for an internal control.

### Example 5

### Shelf life extension of the PCR mixes of the invention

To determine the shelf life of the PCR mixes of the invention, separate tests were performed for the regular thermophilic DNA polymerase (PCR-Ready Mix) and the Hot Start enzyme (PCR-Ready Hot Start) containing mixes.

In a first experiment illustrated in Figure 12A, human genomic DNA (25ng) was amplified using PCR-Ready Mix stored frozen at -200C (F) and stored at Room Temperature (RT) for 98-151 days. Amplicons of 300-1500bp were successfully tested. As shown in Figure 12A, only very mild enzyme activity decay can be observed even after 5 months.

In a second experiment illustrated in Figure 12B, three versions of PCR-Ready Hot Start were compared. Genomic DNA extracted from formalin-fixed paraffin embedded human tissue sample sections was amplified using PCR-Ready Hot Start either (A) freshly prepared; (B) stored at -200C for 60 days or (C) stored at room temperature for 135 days. Amplicons of 200-300bp were successfully tested. The amplicons were chosen based on the application. Mild enzyme activity decay can be observed after 4.5 months. Usually DNA extracted from formalin-fixed paraffin embedded tissue sections is vastly degraded. In our experience, the Bcl-2 amplicon is hardly amplified from these DNA samples and it was used in this experiment as a threshold for quality.

### Example 6

### Requirement for adjusting the ratio balance of various reagent components to facilitate reaction optimization in a PCR duplex using a Regular PCR mix versus a Hydration Reduced PCR mix that will undergo hydration reduction.

As is known to those skilled in the art, adjustments in the ratio balance of the various reagent components of a PCR mix are required to facilitate optimal amplification of each of the assays in a duplex or multiplex PCR reaction. In this example, we demonstrate the novel finding that adjustments in the ratio balance of the various reagent components that would be optimal for a multiplex reaction using a wet PCR mix that will not undergo hydration reduction ("Regular PCR mix"), are not predictive of the adjustments that are required to enable optimal amplification using a PCR mix that undergoes hydration reduction ("Hydration Reduced PCR mix").

In each of the experiments presented in this example, identical quantities of sucrose and BSA were added to both the Regular PCR mix and to the Hydration Reduced PCR mix. The only variable differentiating the Regular PCR mix from the Hydration Reduced PCR mix is the hydration reduction and subsequent rehydration of the mix. Thus, the profound differences in performance of the amplifications that are shown, can be directly attributed to the effects of the mixture undergoing hydration reduction to facilitate extended room temperature shelf life stability and subsequently being rehydrated in preparation for the PCR reaction.

A duplex system was used that expressed the gene products BCL-2 (expressing the human protein B cell CLL/lymphoma 2, product size 291 bp) and GAPDH (encoding the human protein Glyceraldehyde-3-phosphate dehydrogenas, product size 916 bp). In each experiment presented, an identical balance of primer concentrations and DNA template quantity is used both in the Regular PCR mix ("Wet") and the Hydration Reduced PCR mix ("Dry"). A total of three experiments are shown, presenting five different condition sets in which the Regular PCR mix was compared to the Hydration Reduced PCR mix. Adjustments where made in the ratio balance of three reagents components of the mixture in these examples - the concentration of the first set of primers, the concentration of the second set of primers and the amount of template DNA.

**Table 2**

| Experiment number | Name of condition set | Concentration of BCL-2 primers (µM) | Concentration of GAPDH primers (µM) | Amount of template DNA (ng) |
|---|---|---|---|---|
| 1 | A | 0.2 | 0.75 | 1.24 |
| 1 | B | 0.36 | 0.66 | 1.25 |
| 2 | C | 0.38 | 0.75 | 1.15 |
| 3 | D | 0.34 | 0.9 | 1.15 |
| 3 | E | 0.34 | 1.2 | 1.15 |

The Colored hydration reduced PCR Regular Mix was used with 0.8 unit Taq Polymerase enzyme. Sequence of BCL-2 primers is given in Table 1. Sequence of GAPDH primers are: GCCATCAATG ACCCCTTCAT TG (SEQ ID No. 32) and TCTTACTCCT TGGAGGCCAT GT (SEQ ID No. 33). All experimental systems of each experiment were run simultaneously in the same PCR machine.

The results of the three experiments are shown in Figure 13. As evident therein, condition set A of the first experiment resulted in a strong amplification of both amplicons targeted using the Dry PCR mix, while the same condition set using the Wet PCR mix yielded a strong band for the BCL-2 gene, but a much weaker amplification of the GAPDH target. Thus, the optimal balance of primer quantity required for a Hydration Reduced PCR mix, is shown as inadequate for the identical duplex assay using a Regular PCR mix.

On the other hand, the results of amplification using condition sets B shows an optimal amplification of both amplicons using a Regular PCR mix, but complete inability of the condition set to produce simultaneous amplification that could be detected when applied to a Hydration Reduced PCR mix. The results of condition sets C, D and E further demonstrate detectable amplification of both amplicons using a Regular PCR mix and failure to cross the threshold of detectability when the same condition set is applied to the same reagent mix that undergoes a process of hydration reduction (the Hydration Reduced or Dry mix).

In these sets of examples, the optimal balance of reagents required to achieve strong amplification of two different amplicons in a duplex reaction when using a Regular PCR mix was condition set B, which was not appropriate for the Hydration Reduced PCR mix, while the optimal condition set using a Hydration Reduced PCR mix was condition set A, which was not appropriate for the Regular mix.

It is generally assumed that once the balance of reagents is established for optimizing a particular multiplex PCR reaction, those same conditions will again succeed in detecting all of the targeted genes when the same mixture is applied to a subsequent sample of DNA. Accordingly, if the same multiplex reaction mixture is applied to a subsequent sample of DNA and the result is a negative finding of one or more of the targeted genes, the assumed interpretation is that the undetected gene was not present in the sample. As demonstrated in this example, this assumption is not true when attempting to replicate results achieved first using a Regular PCR mix and subsequent using a Hydration Reduced PCR mix. Thus, this example teaches that a Hydration Reduced PCR mix is effective and can be optimized for performing multiplex PCR reactions, but requires that the skilled practitioner disregard the condition set assumptions established for the particular multiplex reaction using a Regular PCR mix and instead readjust the ratio of the various components of the reaction mixture to establish a set of optimal conditions that are unique for performance of the multiplex reaction using a Hydration Reduced PCR mix.

### Example 7

### Requirement for adjusting the ratio balance of various reagent components to facilitate reaction optimization in a Real-Time PCR duplex using a Regular PCR mix versus a Hydration Reduced PCR mix that will undergo hydration reduction

As noted above, it is a teaching of this patent that the mix components in the reaction interact with each other in a different manner during the hydration reduction process of the invention than they do prior to hydration reduction, and that this profoundly influences the performance of a duplex or multiplex reaction assay when a Hydration Reduced PCR mix is later hydrated and used for PCR. While duplex and multiplex Quantitative Real-Time PCR amplification reactions (QRT-PCR) tend to be more specific because of the presence dual-labeled probes and the use of hot start DNA polymerase, the present example demonstrates the finding that adjustments in the ratio balance of the various reagent components that would be optimal for a multiplex reaction using a wet QRT-PCR mix that will not undergo hydration reduction ("Regular QRT-PCR mix"), are not predictive of the adjustments that are required to enable optimal amplification using a QRT-PCR mix that undergoes hydration reduction ("Hydration Reduced QRT-PCR mix").

Example sets containing a duplex assay and a Regular QRT-PCR mix were compared to example sets containing the same duplex assay and a Hydration Reduced QRT-PCR mix. Each set included variable amounts of MgCl2 (reaction final concentrations: 2.5, 4 and 5mM) and variable amount of the primer mixes (reaction final concentrations: 0.15, 0.25, 0.35 and 0.5 µM).

As demonstrated in Example 6 above, the presence or absence of hydration reduction is the key factor in differentiating the component concentration balance optimization requirements of a PCR reaction mix containing a duplex or multiplex assay , and not the presence or absence of stabilizing agents in the reaction mix. Therefore, in the present example, stabilizing agents were only added to the Hydration Reduced QRT-PCR mix and not to the Regular QRT-PCR mix.

The following Real Time Duplex PCR reaction was performed using the set of primers and probes for the human beta-hemoglobin gene and beta 2 microglobulin genes as described below:

**Table 3**

| SEQ ID # | Primer/Probe Sequence (5'→ 3') | Locus Name |
|---|---|---|
| 23 | TGCTGTCTCCATGTTTG | Beta 2 Microglobulin (B2M) |
| 24 | AGTTGCCAGCCCTCCT | |
| 25 | FAM-AGCAGGTTGCTCCACAGGTAGC-BHQ1 | |
| 26 | ACACAACTGTGTTCACTAGC | Beta Hemoglobin (HHB) |
| 27 | CAACTTCATCCACGTTCACC | |
| 28 | HEX-CCACAGGGCAGTAACGGCAGACT-BHQ1 | |

For the Regular QRT-PCR mix, a Hot Start DNA polymerase enzyme was mixed with its buffer (supplied by manufacturer), dNTPs mix (final reaction concentration: 0.2mM), MgCl2 (final reaction concentration: 2.5mM, 4mM or 5mM), B2M and HHB probes (each one final reaction concentration: 0.2µM) and primer mixes (forward and reverse) for each target (each one final reaction concentration: 0.15µM, 0.25µM, 0.35µM or 0.5µM).

| MgCl₂ | 2.5mM | 4mM | 5mM |
|---|---|---|---|
| Primers | 0.15µM | 0.15µM | 0.15µM |
| | 0.25µM | 0.25µM | 0.25µM |
| | 0.35µM | 0.35µM | 0.35µM |
| | 0.5µM | 0.5µM | 0.5µM |

For the Hydration Reduced QRT-PCR mix set, the Clear hydration reduced PCR Hot Start Mix was used with adjustments for the MgC12 and primer concentration as described above.

PCR amplification was performed in a Rotor-Gene 6000 machine (Corbett, Australia) after adding 50ng of human genomic DNA and operating the following cycling: 10-15 min at 95°C, 40-45 repeats of 15sec at 95°C, 20sec at 55°C and 20 sec at 72°C. PCR results were evaluated using the green and yellow channels (for FAM and HEX respectively) and analyzed using the machine software after applying the following parameters: Threshold 0.01; left threshold 10.000; noise slope correction; dynamic tube normalization and No Template Control Threshold 30%.

PCR products were also analyzed after gel electrophoresis on a TBE 3% agarose gel and ethidium bromide staining.

As can be seen in Figure 14, matching MgCl2 and/or primer concentrations affect differently the reaction results depending on the initial hydration grade of the PCR mix. For example, 5mM MgCl2 will promote better results in Regular QRT-PCR mixes (Fig. 14-A) while reduced MgCl2 concentration favors the hydration reduced mixes performance (Fig. 14-C and 15E).

When the MgCl2 concentration was kept the same, Hydration Reduced QRT-PCR mix rendered results even with lower amount of primers (Fig. 14-B, 14-C, 15-C).

The rising amount of primers is reflected by the increasing fluorescent readings as exemplified in figure 16 without affecting the cycle threshold value (Ct) (Figure. 15C).

Figures 16-A and 16-B show Hydration Reduced QRT-PCR mixes incorporating two different brands of DNA polymerase. The results show that a different quantity of MgCl2 was required in the second mix in order to achieve a comparable response to increasing amount of primers in the Hydration Reduced QRT-PCR mix. Therefore, reoptimization of duplex Real Time PCR reactions when switching from a regular QRT-PCR mix to a Hydration Reduced QRT-PCR mix requires differential calibration, depending on the DNA polymerase source.

While dealing with duplex or multiplex PCR reaction amplifications, the first step is to assure the equivalent amplification of the amplicons in the reaction. As illustrated in figure 15, identical MgCl2 and primer concentration, not necessarily will render a double amplification. It is clear from Fig.15-C and 15-E the different outcome when using Regular QRT-PCR mixes or the Hydration Reduced QRT-PCR mix.

Furthermore, even in such cases when both bands are visualized, not necessarily a significantly detectable fluorescent signal would be emitted (Fig. 14, 15-B & 15D). This possibility reflects the non-ideal conditions for the hybridization of the fluorescent label probe to its template. Optimization of the hybridization conditions also was shown to be different in the Regular QRT-PCR and Hydration Reduced QRT-PCR mixes.

It can be concluded that the reagent ratio balance optimization for a Regular QRT-PCR mixes that is not going to be hydration reduced, will not properly performed in the hydrated reduced format, and practical experimentation is needed in order to optimize the mix composition and component concentration in each specific reaction format. Therefore, in contrast to conventional expectation, it is the teaching of this invention that that a Hydration Reduced QRT-PCR mix can be optimized to perform multiplex QRT-PCR reactions, but that the optimal balance of component concentrations is likely to be different from that used to perform the same multiplex QRT-PCR reaction using a QRT-PCR mix that is not going to experience hydration reduction. Accordingly, use of a Hydration Reduced QRT-PCR mix for performance of a duplex or multiplex QRT-PCR reaction requires abandonment of the balance of the components concentrations derived from experimentation for the particular multiplex assay application using a Regular QRT-PCR mix and empirical assessment of said balance of component concentrations using a Hydration Reduced QRT-PCR mix.

### Example 8

### Shelf Life stability of the mix of the invention incorporating a dultiplex Quantitative Real-Time PCR Assay

To establish the performance and shelf life stability of the PCR mixes of the invention for multiplex real-time PCR application, we prepared a duplex primer-probe assay.

One primer-probe set targeting human Beta-Hemoglobin was prepared and purified consisting of forward and reverse primers (SEQ. ID 29 and 30) (Table 4) and a dual labeled probe with HEX fluorescent dye use as reporter, added to the 5'-end and BHQ 1 as quencher added to the 3'-end (SEQ. ID 31) (Table 4). A second primer-probe set targeting the human RNase-P gene was purchases from Applied Biosystems Inc. (TaqMan® RNase P Detection Reagents Kit, Part Number 4316831), consisting of a forward primer, a reverse primer and a TaqMan® Probe labeled with FAM at the 5'-end and TAMRA at the 3'-end.

All four primers and two probes were added to the Clear Hot Start PCR mix of the invention prior to the hydration reduction process. The tubes were stored at room temperature and -200C until testing for assay performance.

After five weeks, a tube stored at room temperature as well as a tube stored at -200C, both containing the dehydrated PCR mix and duplex primer-probe assay were rehydrated and compared to the commercial Absolute QPCR mix (ABgene) mix performance after adding 100 ng of human DNA to each reaction tube. The mixtures were amplified in a RotorGene 6000 System Real Time PCR instrument from Corbett Life Science according to the following protocol: First, a 15min hold cycle at 950C for enzyme activation, followed by 40 cycles consisting of fifteen seconds at 950C, twenty seconds at 550C and thirty seconds at 720C.

The results of the first round of experimentation are illustrated in figure 17-A. The primer-probe assay targeting human Beta-Hemoglobin yielded the following results using the same analysis parameters as described in the previous Example. The Sample that was stored at -200C yielded a Ct of 24.09 and the sample stored at room temperature yielded a Ct of 23.84. The primer-probe assay targeting human RNase-P yielded the following results. The Sample that was stored at -20°C yielded a Ct of 21.18 and the sample stored at room temperature yielded a Ct of 21.52.

The second round of experimentation was conducted five weeks later. The procedure was identical with the exception that three tubes stored at room temperature and one tube stored at -200C were tested in the experiment. In three of the tubes 100 ng of human DNA was added and 150ng DNA was added to the forth tube. Samples were amplified as described above.

The results of the second round of experimentation shown in figure 17-B were as follows. The primer-probe assay targeting human Beta-Hemoglobin yielded the following results. The Sample that was stored at -200C yielded a Ct of 26.26 and the two samples stored at room temperature yielded a Ct of 26.40 and 28.27. The primer-probe assay targeting human RNase-P yielded the following results. The Sample that was stored at -200C yielded a Ct of 23.06 and the two samples stored at room temperature yielded a Ct of 23.79 and 24.34.

After ten weeks, the difference in effectiveness between the stabilized Hydration Reduced mixtures of the invention left sitting out at room temperature and comparable mixtures stored at -200C was less than one cycle. The results clearly demonstrate the utility and stability at room temperature of the PCR mixes of the invention for multiplex Real-Time PCR assays.

**Table 4**

| | |
|---|---|
| SEQ 29 | ACA CAA CTG TGT TCA CTA GC |
| SEQ 30 | CAA CTT CAT CCA CGT TCA CC |
| SEQ 31 | HEX-CCA CAG GGC AGT AAC GGC AGA CT-BHQ1 |
| SEQ 32 | GCCATCAATG ACCCCTTCAT TG |
| SEQ 33 | TCTTACTCCT TGGAGGCCAT GT |

While the invention has been described with reference to particular embodiments, it will be understood by one skilled in the art that variations and modifications may be made in form and detail.

## Claims

1. A method for processing DNA polymerase and/or dNTPs for use in an amplification procedure, the method comprising: providing a solution mixture, the solution mixture including a DNA polymerase and/or dNTPs, a buffer solution and at least one stabilizing agent, wherein said stabilizing agent comprises at least one sugar and at least one protein; and
hydration reducing the solution mixture, wherein the solution mixture is hydration reduced at a temperature between 25°C and 100°C.

2. The method of claim 1 further comprising: storing the hydration reduced solution mixture at ambient room temperature for up to 24 months; rehydrating the stored hydration reduced solution mixture; and performing an amplification procedure using the rehydrated hydration reduced solution mixture.

3. The method of claim 1 wherein the DNA polymerase is a thermophilic DNA polymerase.

4. The method of claim 1 wherein hydration reducing the solution mixture comprises heating the solution mixture in an oven at a temperature of about 55 °C.

5. The method of claim 1 wherein the solution mixture is hydration reduced between 50 percent and 100 percent.

6. The method of claim 1 wherein the sugar comprises a non-reducing sugar and the protein is Bovine Serum Albumin (BSA).

7. The method of claim 6 wherein the non-reducing sugar comprises sucrose, the sucrose in a final concentration range from 1-20% and wherein the BSA concentration range is 0.5-3 mg/ml.

8. The method of claim 1 wherein the solution mixture further comprises any one or more ingredients selected from a group consisting of a set of two oligonucleotide primers, said oligonucleotide primers differing in sequence from each other; magnesium chloride; a water-soluble dye; a nucleic acid template and a fluorescent dye.

9. A kit comprising the processed DNA polymerase and/or dNTPs of claim 1 wherein said kit further comprises a buffer solution, at least one stabilizing agent, magnesium chloride, a set of two oligonucleotide primers, said oligonucleotide primers differing in sequence from each other and an oligonucleotide probe that differs in sequence from said set of two oligonucleotide primers, with or without a nucleic acid template.

10. The kit of claim 9, wherein said kit further comprises:
at least one additional set of oligonucleotide primers, said oligonucleotide primers differing in sequence from each other and being capable of amplifying a region of target DNA that is distinct from the region of target that may be amplified by the first set of oligonucleotide primers, wherein the reagents of the kit are capable of nucleic acid amplification after having been stored at ambient temperature for up to 90 days and subsequently rehydrated.

11. The kit of claim 10 which further includes at least one oligonucleotide probe that differs in sequence from said first and second set of oligonucleotide primers.

12. The kit of claim 10 wherein the reagent components are contained in a single PCR reaction microtube, contained in a PCR reaction microtube that is part of a microtube strip or contained in a well of a multi-well plate prior to the hydration reduction of the reagents being reduced.

13. The kit of claim 12 wherein the kit further includes a water-soluble dye and wherein the amplification process is PCR.

14. A kit comprising the processed DNA polymerase and/or dNTPs of claim 1, wherein said kit further comprises:
a. a first set of two oligonucleotide primers, one or both of which is fluorescent labeled and/or an additional third oligonucleotide that is a labeled probe, said oligonucleotide primers and probe differing in sequence from each other, and able to detect in a quantitative PCR reaction the presence of a unique nucleic acid sequence,
b. a second set of oligonucleotide primers, one or both of which is fluorescent labeled to serve as a probe or in additional to a third oligonucleotide serving as a probe, said second set of oligonucleotide primers and probe differing in sequence from each other, and able to detect in a quantitative PCR reaction the presence of a distinct nucleic acid sequence that is different from the nucleic acid sequence being detected by the first set of primers and probe,
c. a buffer solution containing one or more stabilizing agents; and
d. magnesium chloride,
e. with or without a DNA template to serve as an internal control,
wherein the kit reagents together in a single mixture are capable of nucleic acid amplification activity after having been stored at ambient temperatures for up to 90 days and subsequently rehydrated.

15. A method according to claim 1, wherein said solution mixture has a shelf life of at least 90 days.

## Patentansprüche

1. Eine Methode zur Bearbeitung von DNA-Polymerase und/oder dNTPs zur Anwendung in einem Amplifikationsverfahren, wobei die Methode Folgendes umfasst: Bereitstellen eines Lösungsgemisches, das eine DNA-Polymerase und/oder dNTPs, eine Pufferlösung und mindestens ein Stabilisierungsmittel beinhaltet, worin das genannte Stabilisierungsmittel aus mindestens einem Zucker und mindestens einem Protein besteht; und
Hydratisierung zur Reduktion des Lösungsmittels, wobei das Lösungsgemisch durch Hydratisierung bei einer Temperatur zwischen 25°C und 100°C reduziert wird.

2. Die Methode nach Anspruch 1, die überdies Folgendes beinhaltet: Aufbewahrung des durch Hydratisierung reduzierten Lösungsgemisches über einen Zeitraum von bis zu 24 Monaten bei Raumtemperatur; Rehydratisierung des aufbewahrten, durch Hydratisierung reduzierten Lösungsgemisches; und Durchführung eines Amplifikationsverfahrens unter Verwendung des rehydratisierten, durch Hydratisierung reduzierten Lösungsgemisches.

3. Die Methode nach Anspruch 1, worin es sich bei der DNA-Polymerase um eine thermophile DNA-Polymerase handelt.

4. Die Methode nach Anspruch 1, worin Reduzieren des Lösungsgemisches durch Hydratisierung ein Erhitzen des Lösungsgemisches in einem Ofen bei einer Temperatur von ca. 55°C beinhaltet.

5. Die Methode nach Anspruch 1, worin das Lösungsgemisch durch Hydratisierung um 50 bis 100 Prozent reduziert wird.

6. Die Methode nach Anspruch 1, worin der Zucker aus einem nicht reduzierenden Zucker besteht und das Protein bovines Serumalbumin (BSA) ist.

7. Die Methode nach Anspruch 6, worin der nicht reduzierende Zucker Saccharose beinhaltet, die Saccharose-Endkonzentration zwischen 1 und 20% beträgt und die BSA-Konzentration im Bereich zwischen 0,5 und 3 mg/ml liegt.

8. Die Methode nach Anspruch 1, worin das Lösungsgemisch überdies einen oder mehrere Inhaltsstoffe umfasst, die aus folgender Gruppe ausgewählt werden: einem Satz von zwei Oligonukleotid-Primern, die sich hinsichtlich der Sequenz voneinander unterscheiden; Magnesiumchlorid; einem wasserlöslichen Farbstoff; einer Nukleinsäure-Vorlage und einem fluoreszierenden Farbstoff.

9. Ein Kit, das die bearbeitete DNA-Polymerase und/oder dNTPs aus Anspruch 1 umfasst, bestehend aus einer Pufferlösung, mindestens einem Stabilisierungsmittel, Magnesiumchlorid, einem Satz von zwei Oligonukleotid-Primern, die sich hinsichtlich der Sequenz voneinander unterscheiden, sowie einer Oligonukleotid-Sonde, die sich hinsichtlich der Sequenz vom genannten Satz der beiden Oligonukleotid-Primer unterschiedet, und mit oder ohne Nukleinsäure-Vorlage vorhanden ist.

10. Das Kit nach Anspruch 9, überdies bestehend aus:
mindestens einem zusätzlichen Set von Oligonukleotid-Primern, wobei sich die genannten Oligonukleotid-Primer hinsichtlich der Sequenz voneinander unterscheiden und in der Lage sind, eine Region der Ziel-DNA, die nicht mit der Zielregion, die vom ersten Satz von Oligonukleotid-Primern amplifiziert werden kann, übereinstimmt, wobei die Reagenzien des Kits zur Amplifikation von Nukleinsäuren fähig sind, nachdem sie bei Raumtemperatur bis zu 90 Tag aufbewahrt und danach rehydriert wurden.

11. Das Kit nach Anspruch 10, das überdies mindestens eine Oligonukleotid-Sonde beinhaltet, die sich hinsichtlich der Sequenz vom ersten und zweiten Oligonukleotid-Primer-Satz unterscheidet.

12. Das Kit nach Anspruch 10, worin die Reagenzkomponenten in einem einzelnen PCR-Reaktions-Mikroröhrchen, das vor der Hydratisierungsreduktion der zu reduzierenden Reagenzien Teil eines Mikroröhrchen-Streifens ist oder sich in einer Vertiefung einer Mehrlochplatte befindet, enthalten sind.

13. Das Kit nach Anspruch 12, wobei das Kit überdies einen wasserlöslichen Farbstoff enthält und das Amplifikationsverfahren eine PCR ist.

14. Ein Kit, das die bearbeitete DNA-Polymerase und/oder dNTPs aus Anspruch 1 umfasst, wobei das genannte Kit überdies Folgendes beinhaltet:
a. einen ersten Satz von zwei Oligonukleotid-Primern, von denen einer oder beide fluoreszierend markiert sind, und/oder ein zusätzliches drittes Oligonukleotid, das eine markierte Sonde ist, wobei die genannten Oligonukleotid-Primer und -Sonde sich hinsichtlich der Sequenz voneinander unterscheiden und in der Lage sind, in einer quantitativen PCR-Reaktion das Vorhandensein einer einzigartigen Nukleinsäuresequenz aufzufinden;
b. einen zweiten Satz von Oligonukleotid-Primern, von denen einer oder beide fluoreszierend markiert sind, um als Sonde zu dienen, oder zusätzlich zu einem dritten Oligonukleotid, das als Sonde dient, wobei der genannte zweite Satz von Oligonukleotid-Primern und -Sonde sich hinsichtlich der Sequenz voneinander unterscheiden, und die in der Lage sind, in einer quantitativen PCR-Reaktion das Vorhandensein einer einzigartigen Nukleinsäuresequenz aufzufinden, die sich von der Nukleinsäuresequenz unterscheidet, die vom ersten Primer- und Sondensatz aufgefunden wird;
c. eine Pufferlösung, die ein oder mehrere Stabilisierungsmittel enthält; und
d. Magnesiumchlorid
e. mit oder ohne DNA-Vorlage, um als interne Kontrolle zu dienen,
wobei die Reagenzien des Kits gemeinsam in einem einzigen Gemisch zur Nukleinsäure-Amplifikationsaktivität fähig sind, nachdem sie bei Raumtemperatur bis zu 90 Tage aufbewahrt und danach rehydriert wurden.

15. Eine Methode nach Anspruch 1, worin die Mindesthaltbarkeit des genannten Lösungsgemisches 90 Tage beträgt.

## Revendications

1. L'invention concerne un procédé pour traiter une ADN-polymérase et/ou des dNTP pour une utilisation dans une procédure d'amplification, le procédé comportant la fourniture d'un mélange de solution, le mélange de solution comprenant une ADN polymérase et/ou des dNTP, une solution tampon et au moins un agent stabilisant, ledit agent stabilisant contenant au moins un sucre et au moins une protéine ; et
la réduction par hydratation du mélange de solution, ce dernier étant réduit par hydratation à une température comprise entre 25°C et 100°C.

2. Procédé selon la revendication 1 consistant en outre à ; conserver le mélange de solution réduit par hydratation à température ambiante pendant 24 mois au maximum ; réhydrater le mélange de solution réduit par hydratation conservé ; et effectuer une procédure d'amplification en utilisant le mélange de solution réduit par hydratation réhydraté.

3. Procédé selon la revendication 1, l'ADN-polymérase étant une ADN-polymérase thermophile.

4. Procédé selon la revendication 1, la réduction par hydratation du mélange de solution consistant à chauffer le mélange de solution dans un four à une température d'environ 55°C.

5. Procédé selon la revendication 1, le mélange de solution étant réduit par hydratation à entre 50 pour cent et 100 pour cent.

6. Procédé selon la revendication 1, le sucre contenant un sucre non-réducteur et la protéine étant de l'albumine de sérum bovine (BSA).

7. Procédé selon la revendication 6, le sucre non-réducteur contenant du sucrose, la concentration finale du sucrose se situant dans la plage de 1 à 20% et la concentration en BSA se situant dans la plage de 0,5 à 3 mg/ml.

8. Procédé selon la revendication 1, le mélange de solution contenant en outre l'un quelconque ou plusieurs ingrédients sélectionnés parmi un groupe consistant d'un ensemble de deux amorces oligonucléotidiques, lesdites amorces oligonucléotidiques différant en séquence l'une de l'autre ; de chlorure de magnésium ; d'un colorant soluble dans l'eau ; d'une matrice d'acide nucléique et d'un colorant fluorescent.

9. Kit contenant l'ADN polymérase et/ou les dNTP traité(s) selon la revendication 1, ledit kit contenant en outre une solution tampon, au moins un agent stabilisant, du chlorure de magnésium, un ensemble de deux amorces oligonucléotidiques, lesdites amorces oligonucléotidiques différant en séquence l'une de l'autre et une sonde oligonucléotidique qui diffère en séquence dudit ensemble d'amorces oligonucléotidiques, avec ou sans matrice d'acide nucléique.

10. Kit selon la revendication 9, ledit kit comprenant en outre :
au moins un ensemble additionnel d'amorces oligonucléotidiques, lesdites amorces oligonucléotidiques différant en séquence l'une de l'autre et étant aptes à amplifier une région d'ADN cible qui est distincte de la région de la cible susceptible d'être amplifiée par le premier ensemble d'amorces oligonucléotidiques, les réactifs du kit étant aptes à subir une amplification de l'acide nucléique après avoir été conservés à température ambiante pendant 90 jours au maximum et après avoir été ultérieurement réhydratés.

11. Kit selon la revendication 10, qui contient en outre au moins une sonde oligonucléotidique qui diffère en séquence desdits premier et deuxième ensembles d'amorces oligonucléotidiques.

12. Kit selon la revendication 10, les composants réactifs étant contenus dans un microtube de réaction en chaîne de la polymérase unique, contenus dans un microtube de réaction en chaîne de la polymérase faisant partie d'une bande de microtubes ou contenus dans un puits d'une plaque à puits multiples avant la réduction par hydratation des réactifs en cours de réduction.

13. Kit selon la revendication 12, le kit contenant en outre un colorant soluble dans l'eau et le processus d'amplification étant une réaction en chaîne de la polymérase.

14. Kit contenant l'ADN polymérase et/ou les dNTP traité(s) selon la revendication 1, ledit kit comprenant en outre :
a. un premier ensemble de deux amorces oligonucléotidiques, l'une ou les deux desquelles étant étiquetée(s) comme étant fluorescente(s) et/ou un troisième oligonucléotide additionnel qui est une sonde étiquetée, lesdites amorces oligonucléotidiques et la sonde différant en séquence l'une de l'autre et étant aptes à détecter, dans une réaction en chaîne de la polymérase, la présence d'une séquence d'acide nucléique unique.
b. un deuxième ensemble d'amorces oligonucléotidiques, l'une ou les deux desquelles étant étiquetée(s) comme étant fluorescente(s) afin de servir de sonde ou en supplément d'un troisième oligonucléotide servant de sonde, ledit deuxième ensemble d'amorces oligonucléotidiques et la sonde différant en séquence l'une de l'autre et étant aptes à détecter, dans une réaction en chaîne de la polymérase, la présence d'une séquence d'acide nucléique distincte qui est différente de la séquence d'acide nucléique détectée par le premier ensemble d'amorces et la sonde,
c. une solution tampon contenant un ou plusieurs agents stabilisants ; et
d. du chlorure de magnésium.
e. avec ou sans matrice d'ADN servant de témoin interne,
dans lequel les réactifs du kit réunis ensemble dans un même mélange sont capables d'une activité d'amplification de l'acide nucléique après avoir été conservés à température ambiante pendant 90 jours au maximum et ensuite réhydratés.

15. Procédé selon la revendication 1, ledit mélange de solution ayant une durée de conservation d'au moins 90 jours.
